# EUROPEAN PATENT SPECIFICATION

(11) **EP 0 775 118 B1**
(45) Date of publication and mention of the grant of the patent: **04.06.2003**
(21) Application number: 95917441.8
(22) Date of filing: 18.05.1995
(51) Int. Cl.: C07D 235/06, C07D 235/26, C07D 403/12, A61K 31/445, A61K 31/495

(54) **BENZIMIDAZOLE DERIVATIVES HAVING DOPAMINERGIC ACTIVITY**
BENZIMIDAZOLDERIVATE MIT DOPAMINERGER WIRKUNG
DERIVES DE BENZIMIDAZOLE PRESENTANT UNE ACTIVITE DOPAMINERGIQUE

(30) Priority: 05.08.1994 US 286576; 30.08.1994 US 298005
(43) Date of publication of application: 28.05.1997
(73) Proprietor: PFIZER INC., New York, N.Y. 10017 (US)
(72) Inventor: FARACI, William, S., East Lyme, CT 06333 (US); FLIRI, Anton, F., J., Norwich, CT 06360 (US); O'NEILL, Brian, T., Old Saybrook, CT 06475 (US); SANNER, Mark, A., Old Saybrook, CT 06475 (US); ZORN, Stevin, H., N. Stonington, CT 06359 (US)
(74) Representative: Eddowes, Simon
(86) International application number: IB9500378
(87) International publication number: WO96004250

(56) References cited:
- EP-A- 0 197 840
- EP-A- 0 526 434
- DE-A- 2 017 265
- ARZNEIMITTEL-FORSCHUNG, vol.35, no.1A, 1985, DE pages 272 - 276 K. FRETER ET AL '4-(indolyl-3)-1-(benzimid azolonylalkyl)-piperidines, a novel group of potential antiallergy compounds'
- CHEMICAL ABSTRACTS, vol. 91, no. 19, 5 November 1979, Columbus, Ohio, US; abstract no. 157658x, J. SAWLEWICZ ET AL 'Benzimidazole derivatives. IX. Synthesis of aminoalcohols and thioethers derived from 1-(2-hydroxy-3-chloropropyl)-2-alkylbenzim idazoles' page 633 ; & ACTA POL. PHARM., vol.36, no.1, 1979 pages 17 - 23

## Description

The present invention relates to novel pharmacologically active benzimidazole derivatives and their acid addition salts. The compounds of this invention exhibit central dopaminergic activity and are useful in the treatment of central nervous system (CNS) disorders. They act preferentially on the D4 dopamine receptor.

It is generally accepted knowledge that dopamine receptors are important for many functions in the animal body. For example, altered functions of these receptors participate in the genesis of psychosis, addiction, sleep, feeding, learning, memory, sexual behavior, regulation of immunological responses and blood pressure. Since dopamine receptors control a great number of pharmacological events and, on the other hand, not all of these events are presently known, there is a possibility that compounds that act preferentially on the D4 dopamine receptor may exert a wide range of therapeutic effects in humans.

European Patent Application EP 0526434, which was published on February 3, 1993, referred to a class of substituted benzimidazol-2-ones that contain 1-(aryl and heteroaryl) 4-propyl-piperidine substituents and states that such compounds were found to be centrally acting serotinergic agents. European Patent Application EP 0548813, which was published on June 30, 1993, refers to a class of substituted indole derivatives that contain 1-[3-(4-aryl and heteroaryl) piperazine-1-yl]propyl substituents and states that such compounds were found to be centrally acting serotinergic agents. German Patent Application DE 2017265, which was published on October 15, 1970, refers to a class of substituted 1-[3-(4-phenyl)piperazin-1-yl]propyl-2-methyl-1H-benzimidazoles and states that such compounds were tested in mice and found to have bronchodilating effects. United States Patent 4,200,641, which was issued on April 29, 1980, and German Patent DE 2714437, which was published on May 11, 1989, referto a series of 1-[3-(4-benzhydryl)piperazin-1-yl]propyl-2,3-dihydro-1H-benzimidazol-2-ones. These compounds were tested in mice and found to have antihistaminic activity. United States Patent 4,954,503, which issued on December 31, 1991, refers to a series of indazole derivatives that contain 1-(aryl and heteroaryl) 4-propyl-piperidine substituents and states that such compounds were found to exhibit antipsychotic and analgesic activity in mice.

The benzimidazole and benzimidazolone moiety has been used as a generic substituent in the preparation of a variety of structurally different classes of compounds that exhibit activity on the CNS systems. Examples can be found in Belgium Patent Application BE 904,945, which was published on December 18, 1986, United States Patent 4,954,503, which issued on December 31, 1991, and European Patent Application EP 200,322, which was published on February 28, 1990.

The present inventors have synthesized several substituted 1-[4-(aryl or heteroaryl)-piperazin-1-yl]-3-(2-propyl-benzoimidazol-1-yl)-propan-2-ol; 1-{3-[4-(aryl or heteroaryl)-piperazin-1-yl]-2-hydroxy-propyl}-1,3-dihydro-benzoimidazol-2-one and 1-{3-[4-(aryl or heteroaryl)-piperazin-1-yl]-propyl]-1H-benzoimidazole derivatives that posses central dopaminergic activity. These compounds have a preference for D4-dopamine receptor.

### Summary of the Invention

This invention relates to compounds of the formula 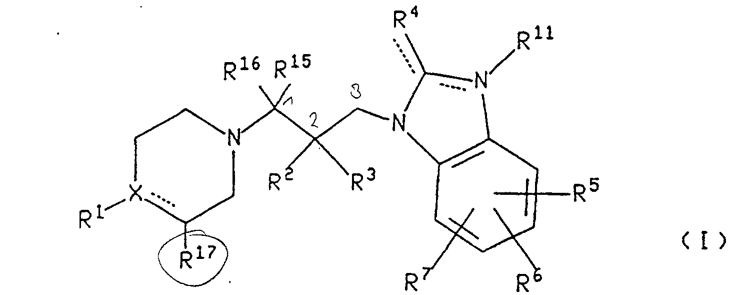 wherein each of the dotted lines represents an optional double bond;
X is carbon or nitrogen;
R¹ is (C₁-C₄) alkyl; aryl; where aryl is selected from phenyl, indanyl and naphthyl; or heteroaryl where heteroaryl is selected from pyridyl, thienyl, furyl, thiazolyl, isothiazolyl, oxazolyl, isoxazolyl, triazolyl, quinolyl and imidazolyl, wherein each of said foregoing (C₁-C₄), alkyl, aryl, and heteroaryl groups may optionally be substituted with one or more substituents independently selected from halo; (C₁-C₆) alkyl optionally substituted with from one to three flourine atoms; (C₁-C₆) alkoxy optionally substituted with from one to three flourine atoms, cyano, -C(=O)R⁸, aryl, wherein said aryl is independently selected from said above-recited aryl groups; and heteroaryl, wherein said heteroaryl is independently selected from said above-recited heteroaryl groups;
R² and R³ are independently selected from hydrogen, hydroxy, and (C₁-C₆) alkyl, provided at least one of R² and R³ is hydroxy;
R⁴ is hydrogen, sulfur, oxygen, (C₁-C₆) alkyl, amino, -NHR¹⁰, -SR¹⁰, or OR¹⁰;
R⁵, R⁶ and R⁷ are independently selected from hydrogen, halo, cyano, (C₁-C₆)alkyl optionally substituted with from one to three fluorine atoms, (C₁-C₆)alkoxy optionally substituted with from one to three fluorine atoms, (C₁-C₆)alkylsulfonyl, (C₁-C₈)acylamino, (phenyl)[(C₁-C₆)acyl]amino, amino, (C₁-C₆)alkylamino, di-(C₁-C₆)alkylamino, aryl and heteroaryl, wherein said aryl is selected from phenyl, naphthyl and indanyl, and said heteroaryl is selected from pyridyl, thienyl, furyl, thiazolyl, isothiazolyl, oxazolyl, isoxazolyl, triazolyl, quinolyl and imidazolyl;
R⁸, R⁹ and R¹⁰ are independently selected from hydrogen and (C₁-C₆)alkyl; and
R¹¹ is hydrogen, (C₁-C₆)alkyl or benzyl, wherein the phenyl moiety of said benzyl may optionally be substituted with one or more substituents, preferably with from zero to two substituents, independently selected from halo, (C₁-C₆)alkyl optionally substituted with from one to three fluorine atoms, (C₁-C₆)alkoxy optionally substituted with from one to three fluorine atoms, amino, cyano, (C₁-C₆)alkylamino and di-(C₁-C₆)alkylamino;
each of R¹⁵ and R¹⁶ is selected, independently, from hydrogen, methyl, cyano, -(C=O)-NH₂ and -CH₂-O-(C₁-C₆)alkyl;
R¹⁷ is hydrogen or, when X is nitrogen, R¹⁷ may optionally form, together with the carbon to which it is attached, R¹ and X, a tetrahydroquinoline ring;

with the proviso that: (a) when the five membered ring of formula I contains a double bond, R¹¹ is absent; (b) when R⁴ is sulfur or oxygen, R⁴ is double bonded to the carbon to which it is attached and such carbon is single bonded to both adjacent ring nitrogen atoms, and when R⁴ is hydroxyl, said carbon is double bonded to one said adjacent ring nitrogen atom as shown by said dotted line; (c) when X is nitrogen and is double bonded to an adjacent carbon, R¹ is absent; and (d) when R⁴ is methyl or oxygen, then both R¹⁵ and R¹⁶ are hydrogen.

The compounds of formula I that are basic in nature are capable of forming a wide variety of salts with various inorganic and organic acids. The acids that may be used to prepare pharmaceutically acceptable acid addition salts of those compounds of formula I that are basic in nature are those that form non-toxic acid addition salts, i.e., salts containing pharmacologically acceptable anions, such as the hydrochloride, hydrobromide, hydroiodide, nitrate, sulfate, bisulfate, phosphate, acid phosphate, isonicotinate, acetate, lactate, salicylate, citrate, acid citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate and pamoate i.e., 1,1'-methylene-bis-(2-hydroxy-3-naphthoate)] salts.

This invention also relates to the pharmaceutically acceptable acid addition salts of compounds of the formula I.

The term "one or more substituents", as used herein, includes from one to the maximum number of substituents possible based on the number of available bonding sites.

The term "alkyl", as used herein, unless otherwise indicated, includes saturated monovalent hydrocarbon radicals having straight, branched or cyclic moieties or combinations thereof.

The term "alkoxy", as used herein, unless otherwise indicated, refers to radicals having the formula -O-alkyl, wherein "alkyl" is defined as above.

Preferred compounds of this invention include the following:
1-Benzoimidazol-1-yl-3-[4-(4-fluoro-phenyl)-piperazin-1-yl]-propan-2-ol;
1-(5-Chloro-benzoimidazol-1-yl)-3-[4-(4-fluoro-phenyl)-piperazin-1-yl]-propan-2-ol;
1-Benzoimidazol-1-yl-3-(4-o-tolyl-piperazine-1-yl)-propan-2-ol;
1-Benzoimidazol-1-yl-3-(4-m-tolyl-piperazine-1-yl)-propan-2-ol;
1-Benzoimidazol-1-yl-3-(4-p-tolyl-piperazin-1-yl)-propan-2-ol;
1-Benzoimidazol-1-yl-3-{4-chloro-phenyl[phenyl-methyl]-piperazin-1-yl}-propan-2-ol;
1-Benzoimidazol-1-yl-3-[4-(2-chloro-phenyl)-piperazin-1-yl]-propan-2-ol;
1-Benzoimidazol-1-yl-3-[4-(4-chloro-phenyl)-piperazin-1-yl]-propan-2-ol;
1-Benzoimidazol-1-yl-3-[4-(3-chloro-phenyl)-piperazin-1 -yl]-propan-2-ol;
1 -Benzoimidazol-1-yl-3-(4-pyrimidin-2-yl-piperazin-1 yl)-propan-2-ol;
1-Benzoimidazol-1-yl-3-(4-naphthalen-1-yl-piperazin-1-yl)-propan-2-ol;
1-Benzoimidazol-1-yl-3-[4-(3-trifluoromethyl-phenyl)-piperazin-1-yl]-propan-2-ol;
1-Benzoimidazol-1-yl-3-(4-benzyl-piperazin-1-yl)-propan-2-ol;
1-Benzoimidazol-1 -yl-3-[4-(2-trifluoromethyl-benzyl)-piperazin-1-yl)-propan-2-ol;
1-Benzoimidazol-1-yl-3-[4-(2-ethoxy-benzyl)-piperazin-1-yl]-propan-2-ol;
1-Benzoimidazol-1-yl-3-{4-{3(3-trifluoromethyl-phenyl)-propyl]-piperazin-1-yl}-propan-2-ol;
1-Benzoimidazol-1-yl-3{4-[2-(3-trifluoromethyl-phenyl)-ethyl]-piperazin-1-yl}-propan-2-ol;
1 -[4-(4-Fluoro-phenyl)-piperazin-1-yl]-3-(2-phenyl-benzoimidazol-1-yl)-propan-2-ol;
1-[4-(4-Fluoro-phenyl)-piperazin-1-yl]-3-(2-propyl-benzoimidazol-1-yl)-propan-2-ol;
1-[4-(4-Fluoro-phenyl)-piperazin-1-yl]-3-(2-methyl-benzoimidazol-1-yl)-propan-2-ol;
5-Chloro-1-(3-[4-(4-fluoro-phenyl)-piperazin-1-yl]-2-hydroxy-propyl)-1,3-dihydrobenzoimidol-2-one;
5-Fluoro-1-(3-[4(4-fluoro-phenyl)-piperazin-1-yl)-2-hydroxy-propyl)-1,3-dihydrobenzoimidazol-2-one;
1-Benzoimidazol-1-yl-3-[4-(3-methoxy-phenyl)-piperazin-1-yl]-propan-2-ol;
1-Benzoimidazol-1-yl-3-[4-(4-methoxy-phenyl)-piperazin-1-yl]propan-2-ol;
1-Benzoimidazol-1-yl-3-(4-phenyl-piperazin-1-yl)-propan-2-ol;
1-{4-[4-(3-Benzoimidazol-1-yl-2-hydroxy-propyl)-piperazin-1-yl]-phenyl}-ethanone;
1-(4-Benzo[1,3]dioxol-5-ylmethyl-piperazin-1-yl)-3-benzoimidazol-1-yl-propan-2-ol;
1-Benzoimidazol-1-yl-3-[4-(3,4-dimethyl-phenyl)-piperazin-1-yl]-propan-2-ol;
4-[4-(3-Benzoimidazol-1-yl-2-hydroxy-propyl)-piperazin-1-yl]-phenol;
1-Benzoimidazol-1-yl-3-(4-phemethyl-piperazin-1-yl)-propan-2-ol;
1-Benzoimidazol-1yl-3-[4-(3-chloro-propyl)-piperazin-1-yl]-propan-2-ol;
2-[4-(3-Benzoimidazol-1-yl-2-hydroxy-propyl)-piperazin-1-yl-1-morpholin-4-yl-ethanone;
1-(4-Benzhydryl-piperazin-1-yl)-3-benzoimidazol-1-yl-propan-2-ol;
1-Benzoimidazol-1-yl-3-{4-[bis-(4-fluoro-phenyl)-methyl]-piperazin-1-yl}-propan-2-ol;
1-Benzoimidazol-1-yl-3-[4-(4-nitro-phenyl)-piperazin-1-yl]-propan-2-ol;
4-(3-Benzoimidazol-1-yl-2-hydroxy-propyl)-piperazine-1-carboxylic;
3- [4-(3-Benzoimidazol-1-yl-2-hydroxy-propyl)-piperazin-1-yl]-1-phenyl-propan-1-one;
4-[4-(3-Benzoimidazol-1-yl-2-hydroxy-propyl)-piperazin-1-yl]-1-(4-fluoro-phenyl)-butan-1-one;
1-Benzoimidazol-1-yl-3-[4-(tetrahydro-furan-2-ylmethyl)-piperazin-1-yl]-propan-2-ol;
[4-(3-Benzoimidazol-1-yl-2-hydroxy-propyl)-piperazin-1-yl]-(tetrahydro-furan-2-yl)-methanone;
[4-(3-Benzoimidazol-1-yl-2-hydroxy-propyl)-piperazin-1-yl]-(2,3-dihydrobenzo[1,4]dioxin-2-yl)-methanone;
1-Benzoimidazol-1-yl-3-[4-(2,3-dihydro-benzo[1,4]dioxin-2-ylmethyl)-piperazin-1-yl]-propan-2-ol;
1-Benzoimidazol-1-yl-3-[4-2-nitro-butyl)-piperazin-1-yl]-propan-2-ol;
3-[4-(3-Benzoimidazol-1-yl-2-hydroxy-propyl)-piperazin-1-yl]-1-(4-chloro-phenyl)-propan-1-one;
1-(5-Fluoro-2-methyl-benzoimidazol-1-yl)-3-[4-(4-fluoro-phenyl)-piperazin-1-yl]-propan-2-ol;

Other embodiments of this invention include:
(a) compounds of the formula I wherein R¹ is phenyl and is either unsubstituted or substituted with one or two substituents selected from halo, (C₁-C₆)alkyl substituted with from one to three fluorine atoms, (C₁-C₆)alkoxy substituted with from one to three fluorine atoms, cyano, -C(=O)R⁸, aryl and heteroaryl;
(b) compounds of the formula I wherein R¹ is indanyl and is either unsubstituted or substituted with one or two substituents selected from halo, (C₁-C₆)alkyl substituted with from one to three fluorine atoms, (C₁-C₆)alkoxy substituted with from one to three fluorine atoms, cyano, -C(=O)R⁸, aryl and heteroaryl;
(c) compounds of the formula I wherein R¹ is naphthyl and is either unsubstituted or substituted with one or two substituents selected from halo, (C₁-C₆)alkyl substituted with from one to three fluorine atoms, (C₁-C₆)alkoxy substituted with from one to three fluorine atoms, cyano, -C(=O)R⁸, aryl and heteroaryl;
(d) compounds of the formula I wherein R¹ is heteroaryl and is either unsubstituted or substituted with one or two substituents selected from halo, (C₁-C₆)alkyl substituted with from one to three fluorine atoms, (C₁-C₆)alkoxy substituted with from one to three fluorine atoms, cyano, C(=O)R⁸, aryl and heteroaryl;
(e) compounds of the formula I wherein R⁵, R⁶ and R⁷ are independently selected from (C₁-C₆)alkyl optionally substituted with from one to three fluorine atoms, (C₁-C₆)alkoxy optionally substituted with from one to three fluorine atoms, cyano and halo;
(f) compounds of the formula I wherein R⁴ is hydrogen;
(g) compounds of the formula I wherein R⁴ is (C₁-C₆)alkyl;
(h) compounds of the formula I wherein R⁴ is amino;
(i) compounds of the formula I wherein R⁴ is -NHR¹⁰;
(j) compounds of the formula I wherein R⁴ is SR¹⁰;
(k) compounds of the formula I wherein R⁴ is -OR¹⁰;
(l) compounds of the formula I wherein R⁴ is hydroxy;
(m) compounds of the formula I wherein R¹¹is absent;
(q) compounds of the formula I wherein one of R² and R³ is (C₁-C₆)alkyl;
(r) compounds of the formula I wherein X is carbon;
(s) compounds of the formula I wherein X is nitrogen;
(t) compounds of the formula I wherein R⁴ is oxygen; and
(v) compounds of the formula I wherein R⁴ is sulfur.

The compounds of formula I above may contain chiral centers and therefore may exist in different enantiomeric forms. This invention relates to all optical isomers and all other stereoisomers of compounds of the formula I and mixtures thereof.

This invention also relates to a pharmaceutical composition for treating or preventing a condition selected from sleep disorders, sexual disorders (including sexual dysfunction), gastrointestinal disorders, psychosis, affective psychosis, nonorganic psychosis, personality disorders, psychiatric mood disorders, conduct and impulse disorders, schizophrenic and schizoaffective disorders, polydipsia, bipolar disorders, dysphoric mania, anxiety and related disorders, obesity, emesis, bacterial infections of the CNS such as meningitis, learning disorders, memory disorders, Parkinson's disease, depression, extrapyramidal side effects from neuroleptic agents, neuroleptic malignant syndrome, hypothalamic pituitary disorders, congestive heart failure, chemical dependencies such as drug and alcohol addictions, vascular and cardiovascular disorders, ocular disorders (including glaucoma), dystonia, tardive dyskinesia, Gilles De La Tourette's syndrome and other hyperkinesias, dementia, ischemia, Parkinson's disease, movement disorders such as akathesia, hypertension and diseases caused by a hyperactive immune system such as allergies and inflammation in a mammal, including a human, comprising an amount of a compound of the formula I, or pharmaceutically acceptable salt thereof, that is effective in treating or preventing such condition, and a pharmaceutical acceptable carrier.

The present invention is useful in a method of treating or preventing a condition selected from sleep disorders, sexual disorders (including sexual dysfunction), gastrointestinal disorders, psychosis, affective psychosis, nonorganic psychosis, personality disorders, psychiatric mood disorders, conduct and impulse disorders, schizophrenic and schizoaffective disorders, polydipsia, bipolar disorders, dysphoric mania, anxiety and related disorders, obesity, emesis, bacterial infections of the CNS such as meningitis, learning disorders, memory disorders, Parkinson's disease, depression, extrapyramidal side effects from neuroleptic agents, neuroleptic malignant syndrome, hypothalamic pituitary disorders, congestive heart failure, chemical dependencies such as drug and alcohol addictions, vascular and cardiovascular disorders, ocular disorders, dystonia, tardive dyskinesia, Gilles De La Tourette's syndrome and other hyperkinesias, dementia, ischemia, Parkinson's disease, movement disorders such as akathesia, hypertension and diseases caused by a hyperactive immune system such as allergies and inflammation in a mammal, including a human, comprising administering to said mammal an amount of a compound of the formula I, or pharmaceutically acceptable salt thereof, that is effective in treating or preventing such condition.

The present invention also relates to a pharmaceutical composition for treating or preventing a condition selected from sleep disorders, sexual disorders (including sexual dysfunction), gastrointestinal disorders, psychosis, affective psychosis, nonorganic psychosis, personality disorders, psychiatric mood disorders, conduct and impulse disorders, schizophrenic and schizoaffective disorders, polydipsia, bipolar disorders, dysphoric mania, anxiety and related disorders, obesity, emesis, bacterial infections of the CNS such as meningitis, learning disorders, memory disorders, Parkinson's disease, depression, extrapyramidal side effects from neuroleptic agents, neuroleptic malignant syndrome, hypothalamic pituitary disorders, congestive heart failure, chemical dependencies such as drug and alcohol addictions, vascular and cardiovascular disorders, ocular disorders (including glaucoma), dystonia, tardive dyskinesia, Gilles De La Tourette's syndrome and other hyperkinesias, dementia, ischemia, Parkinson's disease, movement disorders such as akathesia, hypertension and diseases caused by a hyperactive immune system such as allergies and inflammation in a mammal, including a human, comprising a dopaminergic effective amount of a compound of the formula I, or a pharmaceutical acceptable salt thereof, and a pharmaceutically acceptable carrier.

The present invention is also useful in a method of treating or preventing a condition selected from sleep disorders, sexual disorders (including sexual dysfunction), gastrointestinal disorders, psychosis, affective psychosis, nonorganic psychosis, personality disorders, psychiatric mood disorders, conduct and impulse disorders, schizophrenic and schizoaffective disorders, polydipsia, bipolar disorders, dysphoric mania, anxiety and related disorders, obesity, emesis, bacterial infections of the CNS such as meningitis, learning disorders, memory disorders, Parkinson's disease, depression, extrapyramidal side effects from neuroleptic agents, neuroleptic malignant syndrome, hypothalamic pituitary disorders, congestive heart failure, chemical dependencies such as drug and alcohol addictions, vascular and cardiovascular disorders, ocular disorders (including glaucoma), dystonia, tardive dyskinesia, Gilles De La Tourette's syndrome and other hyperkinesias, dementia, ischemia, Parkinson's disease, movement disorders such as akathesia, hypertension and diseases caused by a hyperactive immune system such as allergies and inflammation in a mammal, including a human, comprising an administering to said mammal a dopaminergic effective amount of a compound of the formula I, or pharmaceutically acceptable salt thereof.

This invention also relates to a pharmaceutical composition for treating or preventing a disease or condition, the treatment or prevention of which can be effected or facilitated by altering (i.e., increasing or decreasing) dopamine mediated neurotransmission in a mammal, including a human, comprising a dopaminergic effective amount of a compound of the formula I, or a pharmaceutically acceptable salt thereof, and a pharmaceutically acceptable carrier.

The invention is also useful in a method of treating or preventing a disease or condition, the treatment or prevention of which can be effected or facilitated by altering (i.e., increasing or decreasing) dopamine mediated neurotransmission in a mammal, including a human, comprising administering to said mammal a dopaminergic effective amount of a compound of the formula I, or a pharmaceutically acceptable salt thereof.

The present invention also relates to a pharmaceutical composition for treating or preventing a condition selected from sleep disorders, sexual disorders (including sexual, dysfunction), gastrointestinal disorders, psychosis, affective psychosis, nonorganic psychosis, personality disorders, psychiatric mood disorders, conduct and impulse disorders, schizophrenic and schizoaffective disorders, polydipsia, bipolar disorders, dysphoric mania, anxiety and related disorders, obesity, emesis, bacterial infections of the CNS such as meningitis, learning disorders, memory disorders, Parkinson's disease, depression, extrapyramidal side effects from neuroleptic agents. neuroleptic malignant syndrome, hypothalamic pituitary disorders, congestive heart failure, chemical dependencies such as drug and alcohol addictions, vascular and cardiovascular disorders, ocular disorders (including glaucoma), dystonia, tardive dyskinesia, Gilles De La Tourette's syndrome and other hyperkinesias, dementia. ischemia, Parkinson's disease, movement disorders such as akathesia, hypertension and diseases caused by a hyperactive immune system such as allergies and inflammation in a mammal, including a human, comprising a D4 receptor binding effective amount of a compound of the formula I, or a pharmaceutical acceptable salt thereof, and a pharmaceutically acceptable carrier.

The present invention is also useful in a method of treating or preventing a condition selected from sleep disorders, sexual disorders (including sexual dysfunction), gastrointestinal disorders, psychosis, affective psychosis, nonorganic psychosis, personality disorders, psychiatric mood disorders, conduct and impulse disorders, schizophrenic and schizoaffective disorders, polydipsia, bipolar disorders, dysphoric mania, anxiety and related disorders, obesity, emesis, bacterial infections of the CNS such as meningitis, learning disorders, memory disorders, Parkinson's disease, depression, extrapyramidal side effects from neuroleptic agents, neuroleptic malignant syndrome, hypothalamic pituitary disorders, congestive heart failure, chemical dependencies such as drug and alcohol addictions, vascular and cardiovascular disorders, ocular disorders (including glaucoma), dystonia, tardive dyskinesia, Gilles De La Tourette's syndrome and other hyperkinesias, dementia, ischemia, Parkinson's disease, movement disorders such as akathesia, hypertension and diseases caused by a hyperactive immune system such as allergies and inflammation in a mammal, including a human, comprising an administering to said mammal a D4 receptor binding effective amount of a compound of the formula I, or pharmaceutically acceptable salt thereof.

This invention also relates to a pharmaceutical composition for treating or preventing a disease or condition, the treatment or prevention of which can be effected or facilitated by altering dopamine mediated neurotransmission in a mammal, including a human, comprising a D4 receptor binding effective amount of a compound of the formula I, or a pharmaceutically acceptable salt thereof, and a pharmaceutically acceptable carrier.

The invention is also useful in a method of treating or preventing a disease or condition, the treatment or prevention of which can be effected or facilitated by altering dopamine mediated neurotransmission in a mammal, including a human, comprising administering to said mammal a D4 receptor binding effective amount of a compound of the formula I, or a pharmaceutically acceptable salt thereof.

The term "dopaminergic effective amount", as used herein, refers to an amount of a compound sufficient to inhibit the binding of dopamine to a dopamine receptor with the effect of altering (i.e., increasing or decreasing) dopamine mediated neurotransmission.

### Detailed Description Of The Invention

The preparation of compounds of the formula I are described below. In the reaction schemes and discussion that follows, X, R¹, through R¹¹, R¹⁵ through R¹⁷ and the dotted lines in all formulae are defined as above.

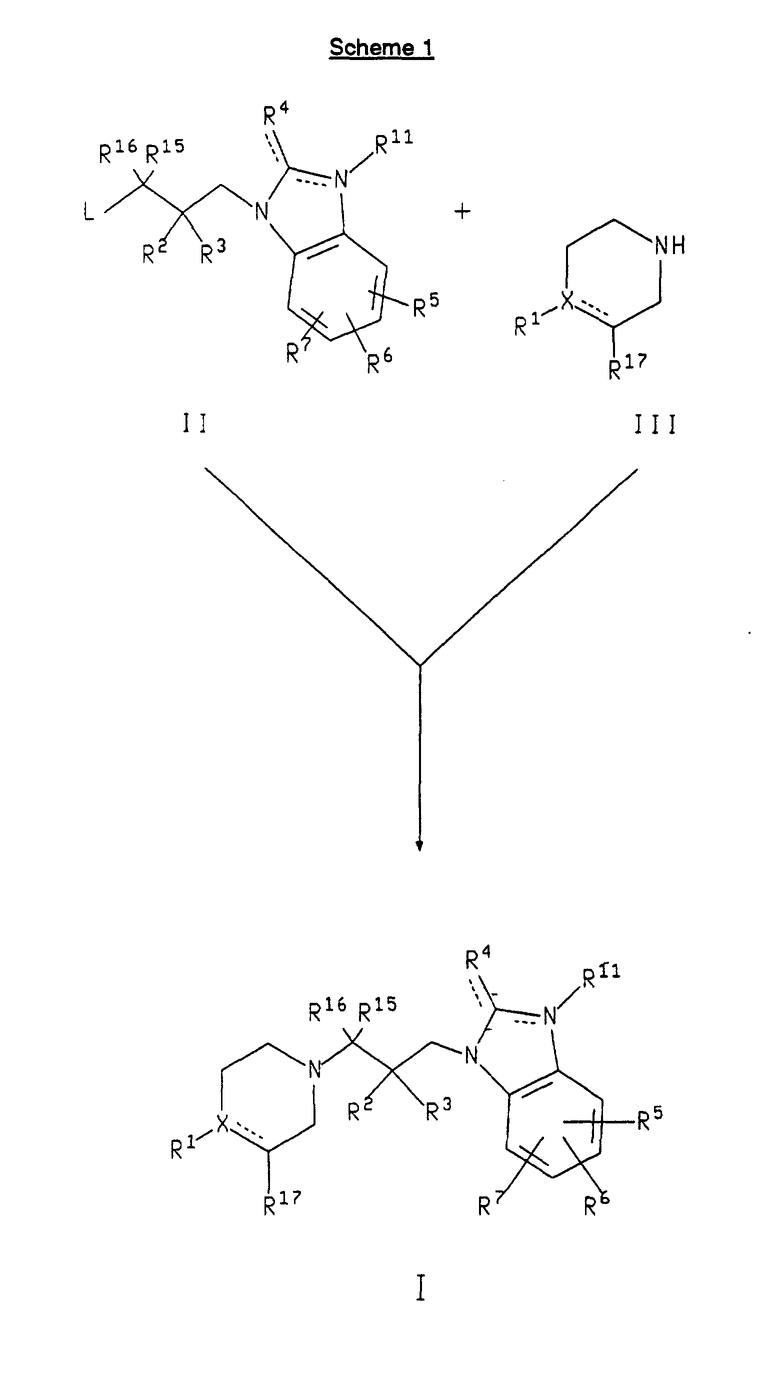

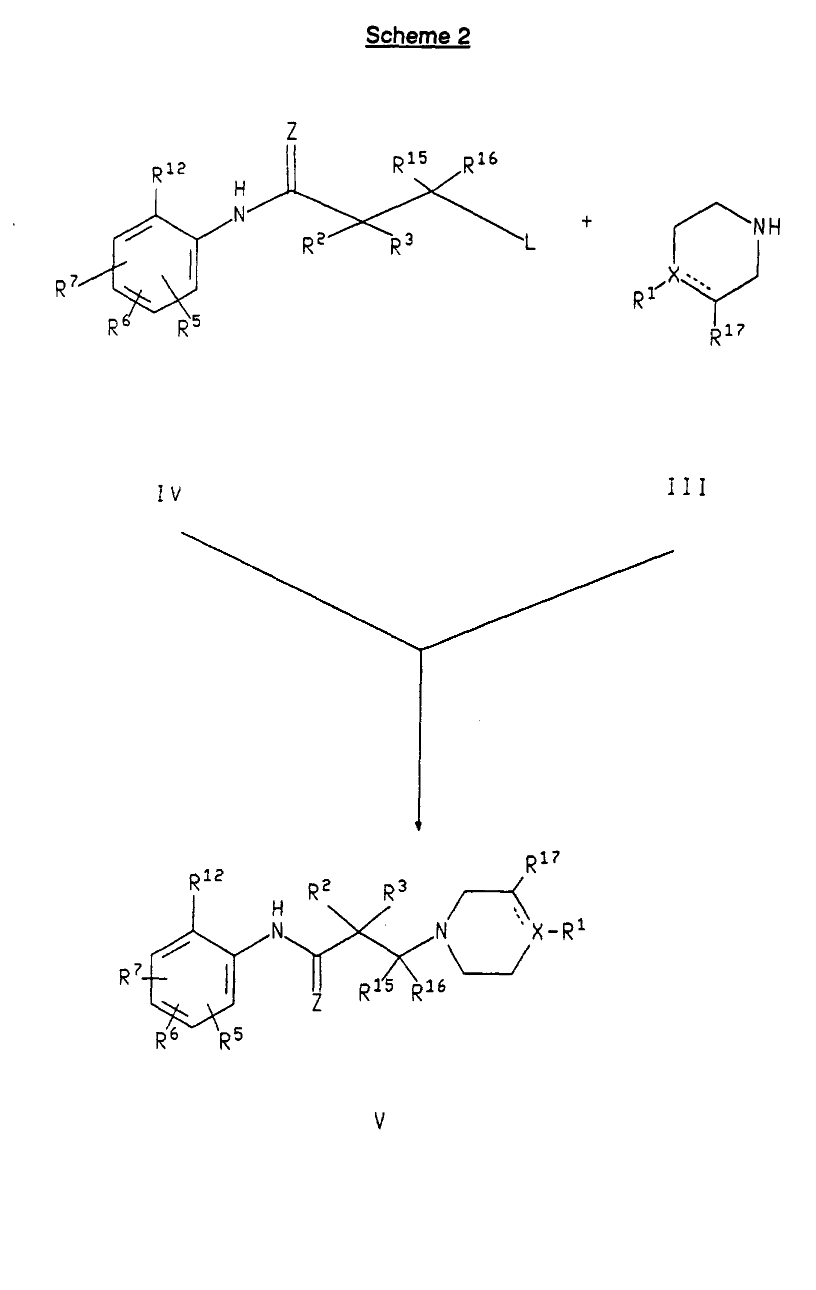

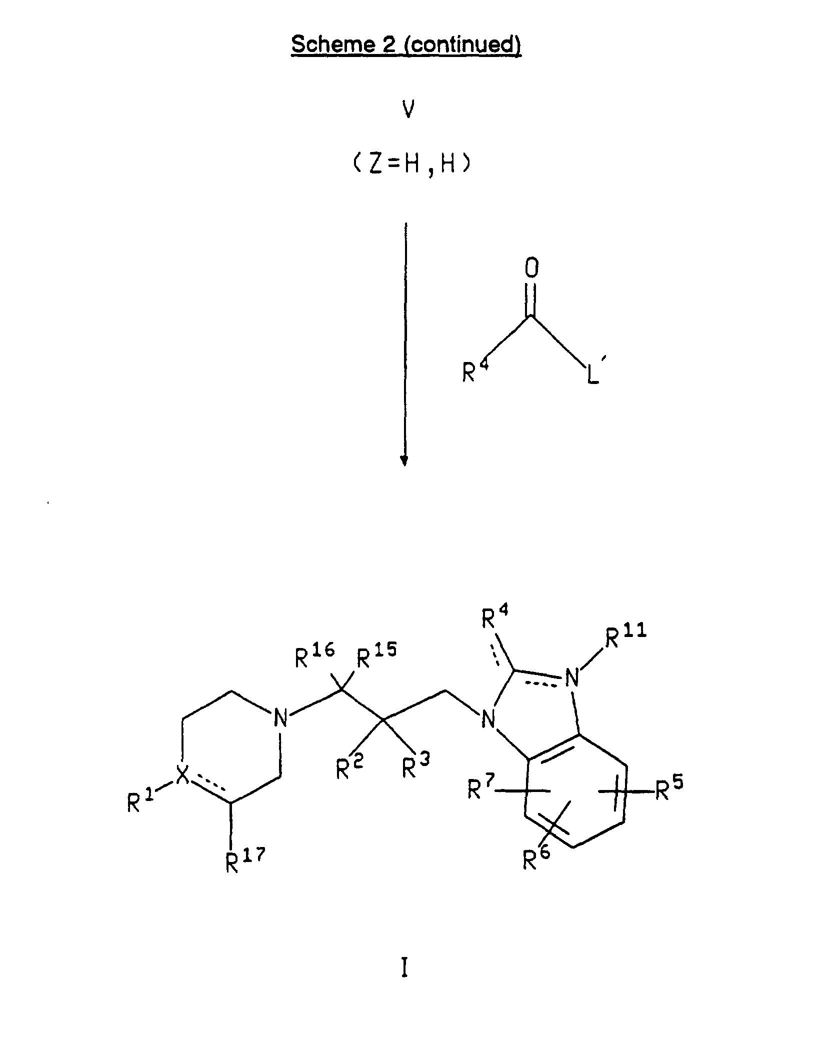

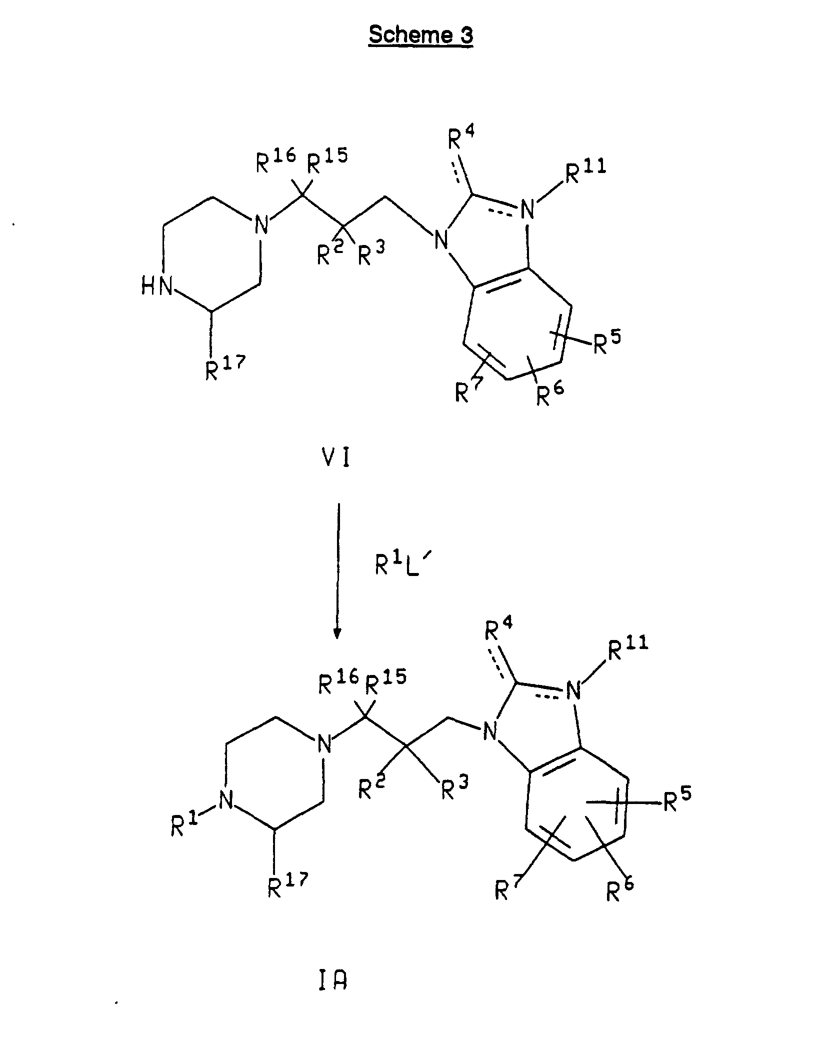

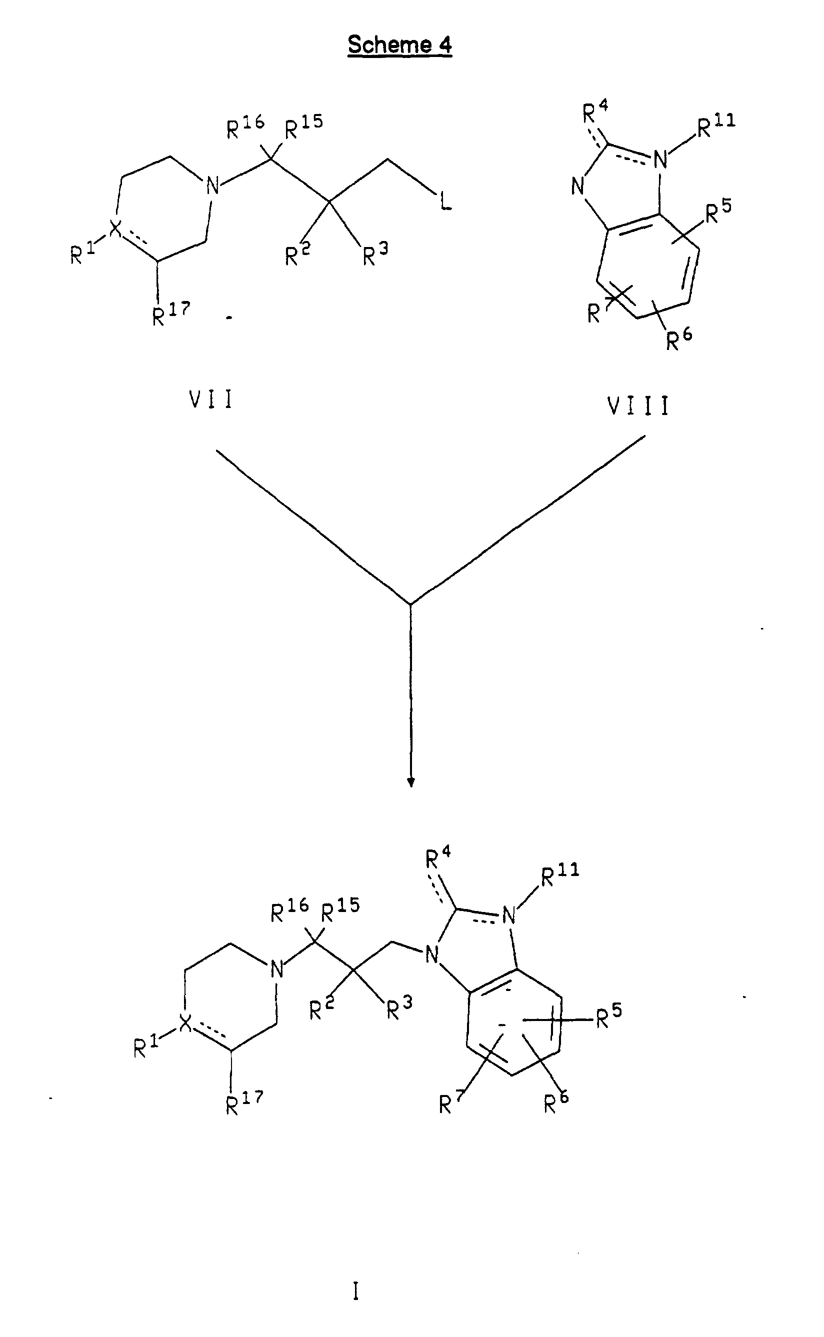

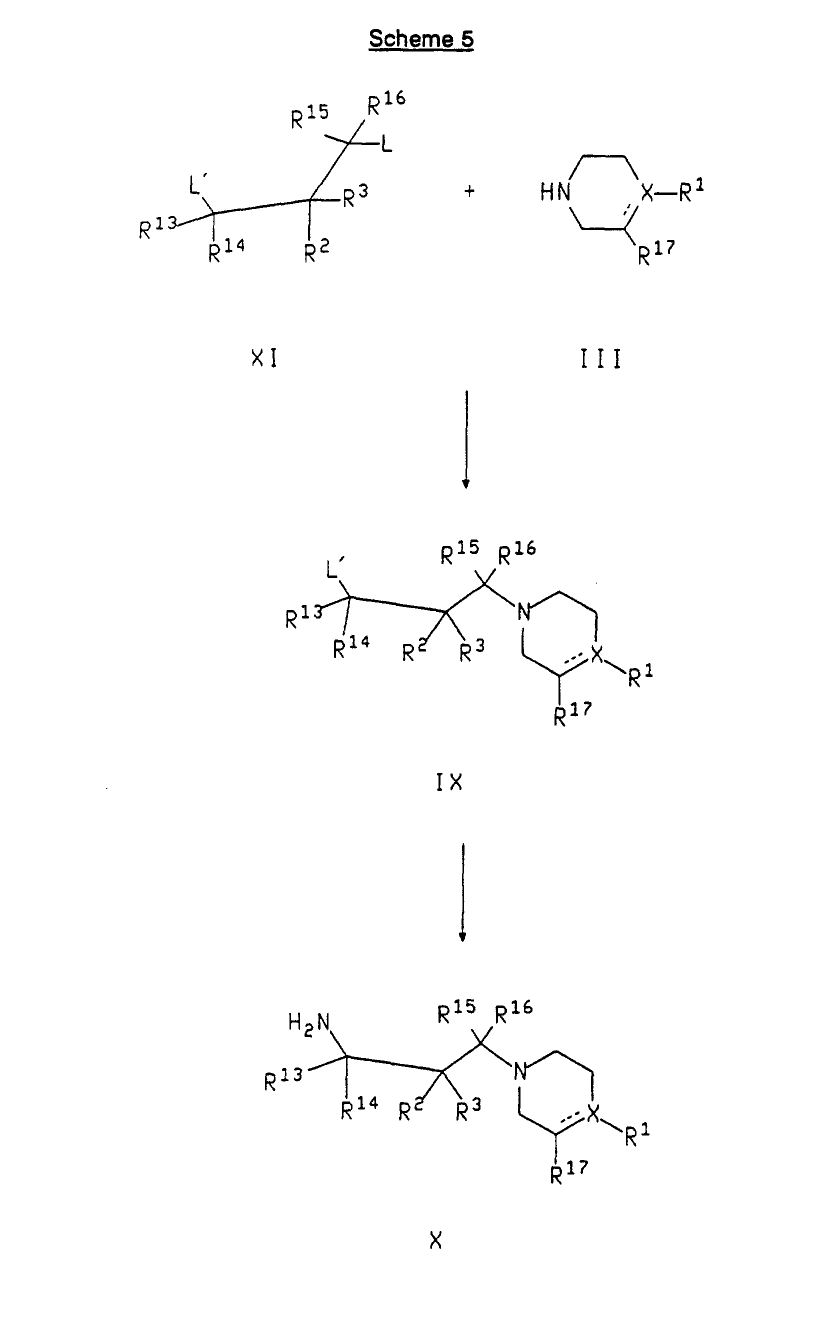

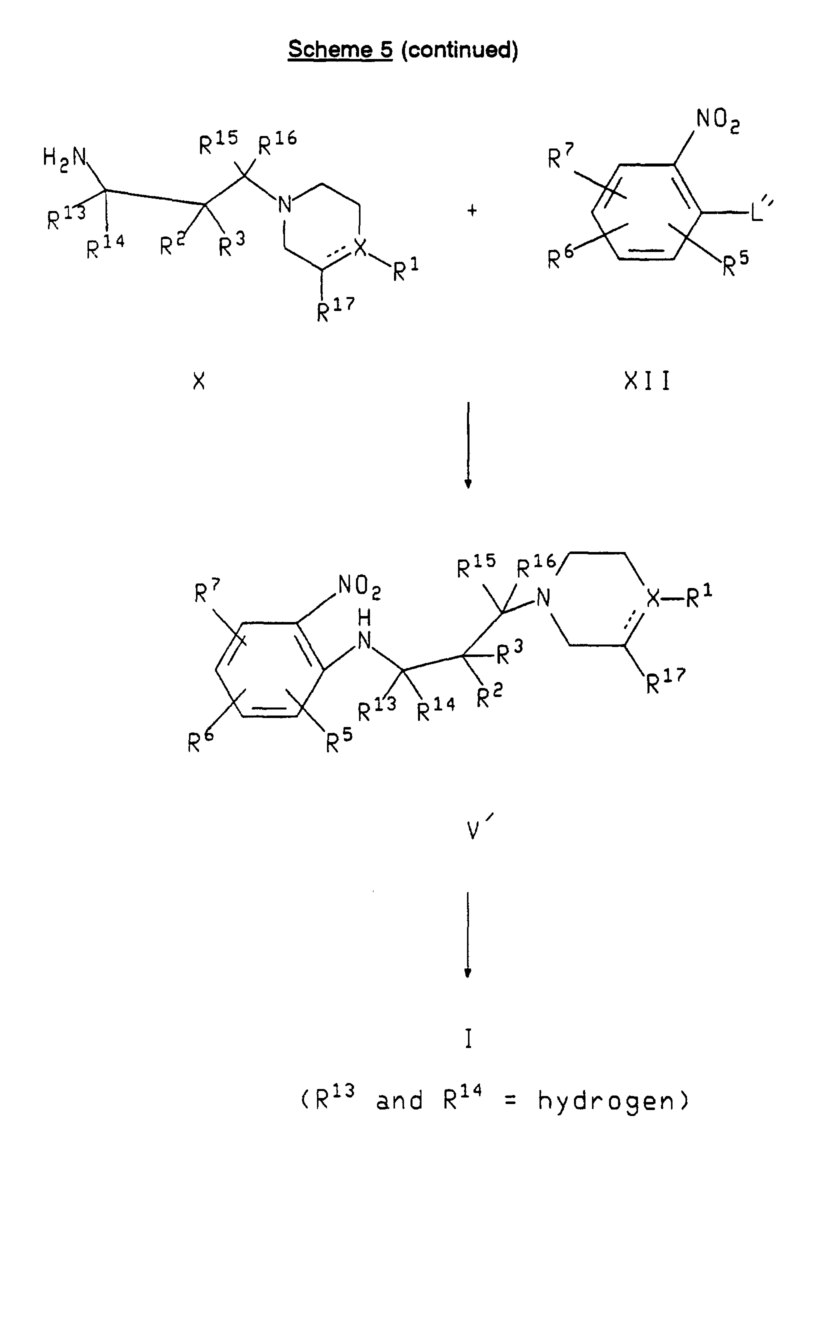

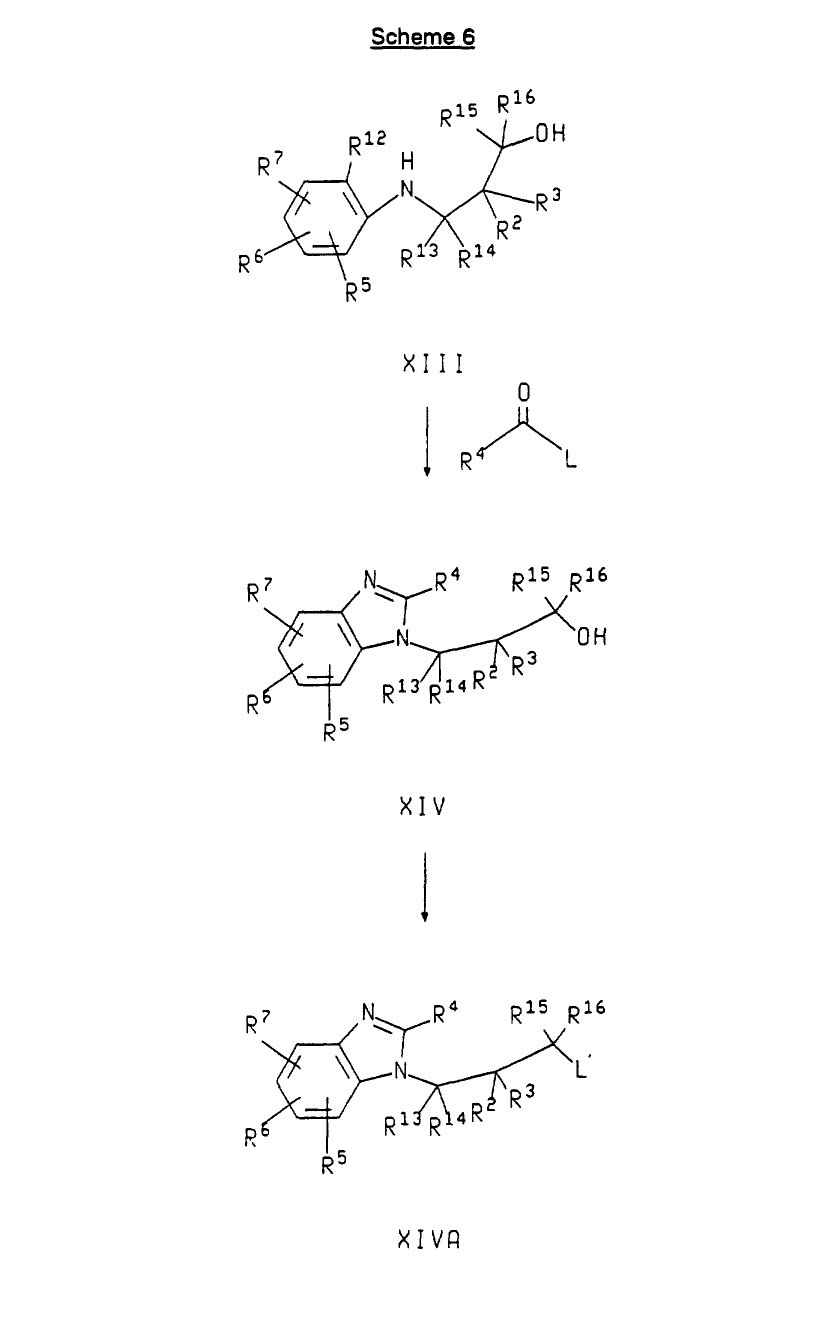

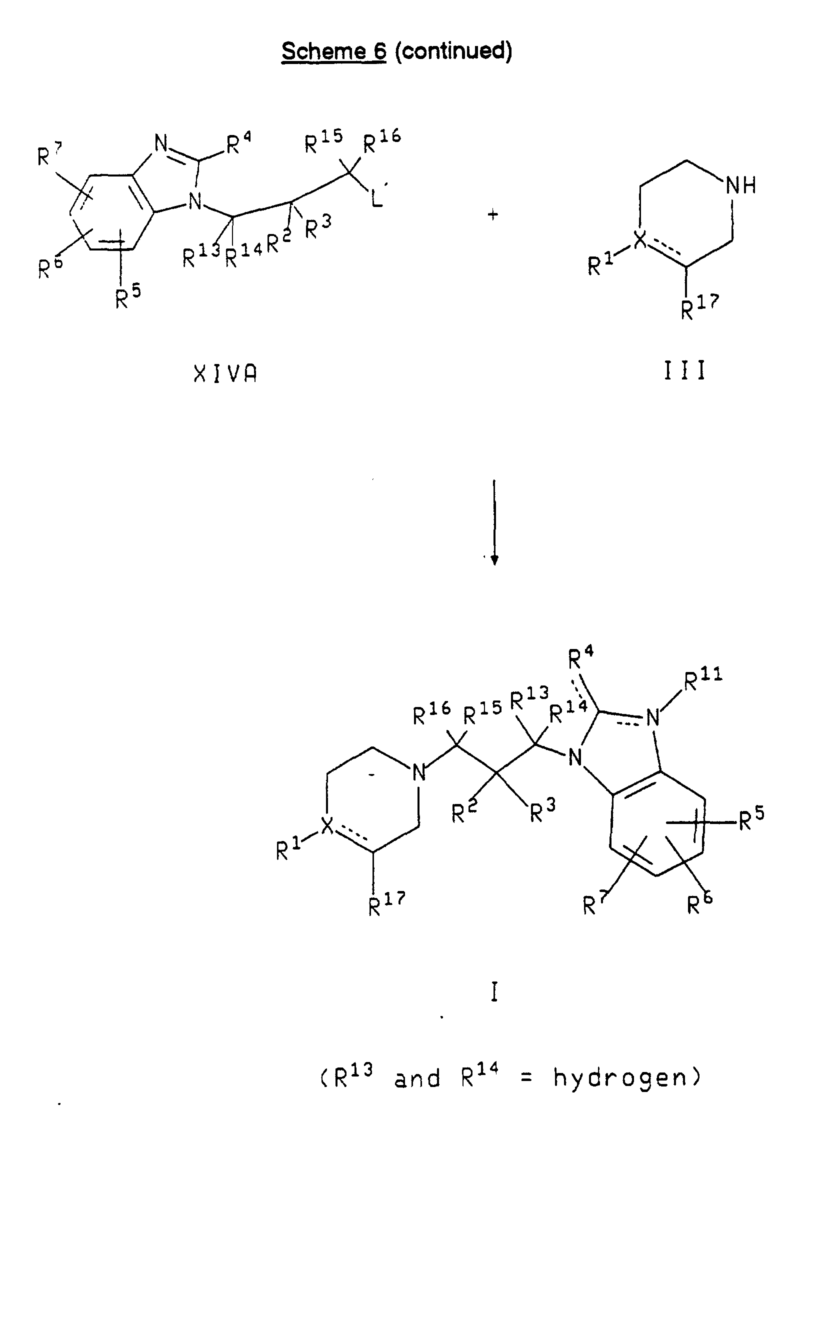

Referring to scheme 1, a compound of the formula II wherein L is a leaving group is reacted with a compound of the formula III to form the corresponding compound of formula I. Suitable leaving groups include chloro, bromo, iodo, -O-(C₁-C₆)- alkylsulfonyl and -O-arylsulfonyl (e.g., -O-phenylsulfonyl, -O-naphthylsulfonyl or -O-paranitrophenylsulfonyl). Also, one of R² and R³ in compound II may be oxygen and may, together with the carbon to which it is attached and L, form an epoxy group, while the other of R² and R³ in such a case is selected from the values given above for these substituents. This reaction is generally carried out in an inert polar solvent such as a lower alcohol, a cyclic or acyclic alkylketone (e.g., ethanol or acetone), an alkylester (e.g., ethylacetate), a cyclic or acyclic mono or dialkylamide (e.g., N-methylpyrdidin-2-one or dimethylformamide (DMF)), a cyclic or acyclic alkyl ether (e.g., tetrahydrofuran (THF) or diisopropyl ether) or a mixture of two or more of the foregoing solvents, at a temperature from about 0°C to about 150°C. It is preferably carried out in ethanol at a temperature from about 0°C to about the reflux temperature of the solvent. The presence of an acid acceptor such as an alkali carbonate or tertiary amine may be useful.

Referring to scheme 2, compounds of the formula I may also be prepared in the following manner. A compound of the formula IV wherein R¹² is selected from NO₂, NH₂, ureido, thiouriedo and -NH(C=O)-Q, wherein Q is hydrogen, (C₁-C₄)alkyl, aryl or heteroaryl, wherein aryl and heteroaryl are defined as in the definition of R¹ above, Z represents one oxygen atom or two hydrogen atoms (each single bonded to the carbon to which Z is attached) and L represents a suitable leaving group, as defined above, is reacted with a compound of the formula III to form an intermediate compound of the formula V. This reaction is typically carried out in an inert polar solvent such as those described above for the reaction of scheme 1, at a temperature from about 0°C to about 150°C. The preferred solvent is ethanol and the preferred temperature is from about 0°C to about the reflux temperature of the solvent. As with the reaction of scheme 1, the addition of acid acceptors such as alkali carbonates and tertiary amines may be useful.

Intermediates of the formula V wherein Z is oxygen must be converted into the corresponding compounds wherein Z is (H, H). This can be accomplished using any of several standard methods of reduction that are well known in the art, e.g., by reacting the compound of formula V wherein Z is oxygen with a solution of lithium aluminum hydride in THF (preferably containing five molar equivalents) in a THF solvent.

The intermediate of formula V wherein Z is (H, H) may be converted, either in situ or after isolation, into the corresponding compound of formula I by reacting it with a compound of the formula R⁴-C(=O)-L' wherein L' is an appropriate leaving group (e.g., chloro, bromo, iodo, fluoro, amino, -O-(C₁-C₆)alkylsulfonyl or O-arylsulfonyl, wherein aryl is selected from phenyl, naphthyl and paranitrophenyl) and an optional dehydrating reagent. Suitable solvents for this reaction include inert polar solvents such as cyclic and acyclic alkyl ethers (e.g., diisopropyl ether and THF), alkylesters (e.g., ethylacetate), cyclic and acyclic alkylketones (e.g., ethanol and acetone), pyridine derivatives (e.g., lutidine and collidine), halogenated solvents (e.g., methylene chloride and dichloroethane) and cyclic, acyclic N-,N-dialkyl alkylamides (e.g., DMF and N-methyl-2-pyrrolidinone (NMP), and acyclic alkylamides (e.g., formamide or acetamide). The reaction temperature may range from about 0°C to about 150°C. Preferably, the reactants (V and R⁴-C(=O)-L') are initially reacted at about 0°C and slowly brought to about the reflux temperature of the reaction mixture. The addition of acid acceptors such as alkali carbonates and tertiary amines may be useful. Addition of a dehydrating agent may also be useful.

Scheme 3 illustrates a method of preparing compounds of the formula I wherein X is nitrogen and R¹ is other than hydrogen. These compounds are referred to in scheme 3 and hereinafter as "compounds of the formula IA." Referring to scheme 3, such compounds can be prepared by reacting the corresponding compounds in which R¹ is hydrogen, with a compound of the formula R¹L' wherein R¹ is other than hydrogen and L' is a suitable leaving group, as defined above. Suitable solvents for this reaction include cyclic and acyclic mono and dialkylamides (e.g., DMF or N-methyl-2-pyrrolidinone), and lower alcohols, and mixtures of two or more solvents from the foregoing classes. Ethanol and N-methyl-2-pyrrolidinone are preferred solvents. The reaction temperature may range from about 0°C to about 150°C, and is preferably between about 0°C and the reflux temperature of the solvent. Addition of an acid acceptor such as an alkali metal carbonate or a tertiary amine may be useful.

An alternate method of preparing compounds of the formula I is illustrated in scheme 4. Referring to scheme 4, a compound of the formula VII, wherein L is a leaving group, is reacted with a compound of the formula VIII to yield a compound of the formula I. Examples of appropriate leaving groups are chlorine, bromino, iodo, -O-(C₁-C₆)alkylsulfonyl and -O-arylsulfonyl wherein aryl may be, for example, phenyl, naphthyl or paranitrophenyl. Also, one of R² and R³ in formula VII can be oxygen and may form, together with the carbon to which it is attached and L, an epoxy group. In such a case, the other of R² and R³ is selected from the values given above in the definitions of these substituents. This reaction is generally carried out using the similar solvents and under similar conditions to those described above for the reaction of scheme 3.

Compounds of the formula II are either commercially available or can be prepared by methods well known to those skilled in the art from compounds of the formula IV. The preparation of a compound of the formula II from a compound of the formula IV is exemplified in Example 2.

Compounds of the formula IV can be prepared by procedures similar to those described in the following literature references: J. Org. Chem., 38, 3498-502 (1973) and J. Chem. Soc. (C), 2364-66 (1971). The synthesis of compounds of the formula IV may be accomplished, for example, by reacting a known aniline derivative containing an R¹² substituent in the ortho position with a substituted or unsubstituted propyl group transferring agent. Such a synthesis is described in Example 8.

Compounds of the general formula III where X is nitrogen may be prepared by reacting commercially available piperazine with aryl transferring reagents such as, for example, 4-nitro fluorobenzene, 2-nitro fluorobenzene or similar reagents followed by well known procedures allowing the exchange of the nitro group against other substituents. Compounds of the general formula III where X is nitrogen may also be prepared by reacting commercially available piperazine with heteroaryl transferring reagents such as, for example, 2-chloro or 2-S-methyl mercapto pyrimidine derivatives, 2-chloro- or 2-bromo pyridine derivatives, 2-chloro or 2-fluoro pyridazine derivatives, 3-chloro isobenzothiazole derivatives, 3-chloro isobenzooxazole derivatives, 3-chloro indazole derivatives or similar reagents. These reactions are preferably carried out in the form of mixtures containing, if desired, combinations of cyclic and acyclic mono and dialkylamides and (C₁-C₄) alcohols or inert organic solvents such as cyclic and acyclic alkyl ethers (e.g., diethyl ether and THF), cyclic and acyclic alkyl esters (e.g., ethylacetate and gama butyrolactones), cyclic and acyclic alkylketones (e.g., acetone and cyclohexanone), pyridine derivatives or halogenated solvents, at temperatures ranging from about 0°C to about 150°C, preferable at a temperature from about 0°C to about the reflux temperature of the reaction mixture. Addition of acid acceptors such as an alkali carbonates, tertiary amines or similar reagents, as well as the addition of dehydrating reagents, may be useful.

Compounds of the formula III wherein X is carbon may be prepared by reacting commercially available 4-piperidinone with aryl transferring reagents such as, for example, aryl grignards or similar reagents and by dehydrating the corresponding benzyl alcohol intermediates. Compounds of the general formula III wherein X is CH may be prepared by reacting commercially available 4-piperidinone with aryl transferring reagents such as, for example, aryl grignards or similar reagents and by hydrogenating the corresponding benzyl alcohol intermediates with either platinum dioxide or palladium on carbon.

Compounds of general structure VI may be prepared by reacting compounds of the general formula II with piperazine or 1-t-butoxycarbonyl piperazine, as exemplified in Example 9, preferably in the form of a mixture containing combinations of (C₁-C₄) alcohols, cyclic and acyclic mono and dialkylamides or inert organic solvents such as cyclic and acyclic alkyl ethers, cyclic and acyclic alkyl esters, cyclic and acyclic alkylketones, pyridine derivatives or halogenated solvents at temperatures ranging from about 0°C to about 150°C, preferable from about at 0°C to the reflux temperature of the solvent mixture. Addition of acid acceptors such as an alkali carbonates, tertiary amines or similar reagents may be useful. Corresponding piperazine intermediates (having different substitution pattern) can be formed by reacting the products of the foregoing reaction with an appropriate aryl transferring reagent such as, for example, 4-nitro fluorobenzene, 2-nitro fluorobenzene or similar reagents followed by well known procedures allowing the exchange of the nitro grouping against other substituents. Such corresponding piperazine intermediates can also be prepared by reacting other intermediates with heteroaryl transferring reagents such as for example, 2-chloro or 2-S-methyl mercapto pyrimidine derivatives, 2-chloro or 2-bromo pyridine derivatives, 2-chloro or 2-fluoro pyridazine derivatives, 3-chloro isobenzothiazole derivatives, 3-chloro isobenzooxazole derivatives, 3-chloro indazole derivatives or similar reagents. The preferred conditions for these reactions are similar to those described in Example 11.

Similarly, compounds of the formula VII may be prepared by reacting compounds of the formula III with compounds of the general formula L²-CH₂-CH(R²)CH(R³)-CH₂-L³, wherein each of L² and L³ is a leaving group that is, for example, independently selected from chloro, bromo, iodo, O-alkylsulfonyl and O-arylsulfonyl, wherein aryl may be, for example, phenyl, naphthyl or paranitrophenyl. Also, one of R² and R³ in the above compound may be oxygen and may, together with the carbon to which it is attached and L² or L³, form an epoxy group, while the other of R² and R³, in such a case, is selected from the values given above for those substituents. The reaction mixture may contain one or more of inert organic solvents such as cyclic and acyclic alkyl ethers, cyclic and acyclic alkyl esters, cyclic and acyclic alkylketones, pyridine derivatives, halogenated solvents or cyclic and acyclic N-,N-dialkylalkylamides. The reaction temperature may range from about 0°C to about 150°C. The reaction is preferable carried out at a temperature from about 0°C to about the of the reflux temperature of the reaction mixture. Addition of an acid acceptor such as an alkali carbonate, a tertiary amine or a similar reagent may be useful.

Compounds of the formula VIII may be prepared according to methods known in the literature, such as, for example, as described in J. Chem. Soc., p. 1396 (1949), Synth. Commun., 5, p. 461 (1975), J. Amer. Chem. Soc., 73, p. 4297 (1951) or J. Chem. Soc., 39, p. 3155 (1951).

Scheme 5 illustrates an alternate procedure for preparing compounds of the formula I. Referring to scheme 5, compounds of the formula IX may be prepared according to methods known in the literature, such as, for example, as described in the following references: J. Chem. Soc., pp. 1396 (1949); Synth. Commun., 5, pp. 461 (1975); J. Amer. Chem. Soc., 73, pp. 4297 (1951); and J. Chem. Soc., 39 pp. 3155 (1951). They may also be prepared, as shown in scheme 5, by reacting an otherwise optionally substituted C₃-C₄ alkyl derivative of the formula Xl with a compound of the formula III. In structure XI, L and L' are defined as above and L' may optionally be a nitro group or a protected amino group when R¹³ and R¹⁴ are both hydrogen; R² and R³ are defined as above and one of R² and R³ may be oxygen and may, together with the carbon to which it is attached and either L or L', form an epoxy group; and R¹³ and R¹⁴ are both hydrogen except that the -CL'R¹³R¹⁴ moiety may optionally be a cyano group (i.e., wherein L' is nitrogen and R¹³ and R¹⁴ represent bonds rather than radicals).

These reactions can be carried out conveniently in solvents such as alcohols, cyclic and acyclic alkylketones, cyclic and acyclic alkylesters, cyclic and acyclic mono and dialkylamides, acetonitrile and cyclic and acyclic alkyl ethers, or mixtures of such solvents, at a temperature from about 0°C to about 150°C, preferably about 0°C or the reflux temperature of the solvent. The presence of an acid acceptor such an alkali carbonate, tertiary amine or a similar reagent may be useful.

For all structures depicted in schemes 5 and 6, R¹³ and R¹⁴ are defined as above. Thus, for all such structures that do not contain the moiety CL'R¹³R¹⁴, both R¹³ and R¹⁴ are hydrogen.

Alternatively, compounds of the general formula IX can be prepared from compounds of the general formula XI, as defined above, by reacting the latter compounds with a compound of the formula 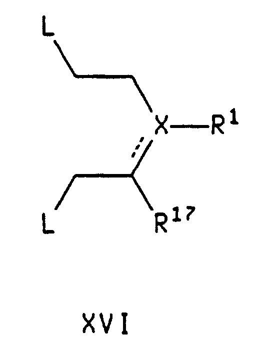 wherein L is defined as above. Appropriate and preferred solvents and conditions for this reaction are similar to those described above or the reaction of compounds of the formulae XI and III.

Compounds of the formula XVI can be prepared by a procedures analogous to that of Example 23A.

Compounds of the formula IX can be converted into the corresponding compounds of formula X as follows. When L' is a protected amino group, (for example, when the nitrogen protecting group is benzyl, benzyloxycarbonyl, t-butoxycarbonyl or trityl), the protecting group can be removed using either hydrogenation or acidic deprotection conditions. When L' is an amino group protected by a phthalimido group, the protecting group may be conveniently removed using standard hydrogenation conditions. When L' is a nitro group, the compound of formula X may be formed by reducing the compound of formula IX using conventional reduction methods. For example, the reduction can be accomplished using a hydride reagent such as lithium aluminum hydride or borane, or using hydrogen gas in the presence of catalyst such as Raney nickel, platinum oxide, palladium/carbon or another appropriate catalyst.

When the -CL'R¹³R¹⁴ moiety is a cyano group, it can be reduced to a -CH₂NH₂ group using, for example, a hydride reagent such as lithium aluminum hydride or sodium borohydride in the presence of cobalt chloride, or using other conventional methods known to those skilled in the art.

Reaction of the resulting compound of the formula X with a compound of the formula XII, wherein L" is defined as L' above, as shown on the second page of scheme 5, yields a compound of the formula V wherein R¹² is nitro (referred to in scheme 5 and hereinafter as a compound of the formula V'), which can then be converted into the corresponding compound of the formula I wherein R⁴ is methyl or trifluoromethyl by reducing the nitro group. This can be accomplished using a metal such as zinc or tin in acetic acid or trifluoroacetic acid, containing, if desired, the corresponding acid anhydride. In the case of compounds of the formula V' containing hydroxy substituents, an ester intermediate may be formed, which can then be converted into the corresponding hydroxy substituted compound of the formula I using conventional methods known to those skilled in the art. For example, such esters can be treated with an aqueous alkali hydroxide solution in an appropriate solvent selected from cyclic and acyclic mono and dialkylamides and C₁-C₄ alcohols and mixtures thereof at a temperature from about 0°C to about 150°C, preferably from about 0°C to about the reflux temperature. The hydroxy substituted compounds of the formula I can be converted into the corresponding alkoxy substituted compounds by treating them first with an alkali hydride such as sodium hydride or calcium hydride, using solvents and conditions as described immediately above, and then with an alkylating agent such as, for example, methyl iodide, dimethylsulfate, allyl iodide, ethyl iodide or a similar reagent. Scheme 6 illustrates the preparation of compounds of the formula I starting with the hydroxy derivatives of formula XIII. The starting materials of formula XIII wherein R¹² is nitro may be prepared by reacting a compound of the formula XI wherein L is hydroxy and L' is amino with a compound of the formula XII, as defined above and depicted in scheme 5. This reaction is generally carried out using similar solvents and conditions to both specified for the reaction of scheme 1.

Compounds of the formula XIII wherein R¹² is nitro may be converted into the corresponding compounds of the formula XIII wherein R¹² is amino using conventional reduction methods such as, for example, using a mixture of a metal such as zinc or tin and hydrochloric acid or, alternatively, using a hydride donating reagent such as, for example, lithium aluminum hydride or borane, or hydrogenating the reactant of formula XIII in the presence of a catalyst such as Raney nickel, platinum oxide or palladium/carbon.

Compounds of the formula XIII wherein R¹² is nitro can be converted into the corresponding compounds of formula XIV wherein R⁴ is methyl or trifluoromethyl by reducing the nitro group with a metal such as zinc or tin in acetic acid or trifluoroacetic acid containing, if desired, the corresponding acid anhydride. This reaction produces an ester intermediate which can then be converted into compounds of the formula XIV using standard methods known to those skilled in the art. For example, the esters can be treated with an aqueous alkali hydroxide solution in an appropriate solvent selected from cyclic and acyclic mono and dialkylamides and C₁-C₄ alcohols, at temperatures from about 0°C to about 150°C, preferably from about 0°C to about the reflux temperature.

Alternatively, compounds of the formula XIV wherein R⁴ is an alkyl group may be prepared by reacting the appropriate compounds of the formula XIII wherein R¹² is amino with an acylating agent such as propionyl chloride, isopropionyl bromide, acetic acid/formic acid anhydride, ethyl formate or a similar reagent, and then treating the intermediary product formed with an aqueous solution of an acid such as hydrochloric acid, trifluoroacetic acid or methanesulfonic acid, preferably hydrochloric acid at a temperature ranging from about 0°C to about the boiling point of the acid.

Compounds of the formula XIV wherein R⁴ is methyl or trifluoromethyl may be converted into compounds of the formula XIVA wherein L' is a leaving group such as chloro, bromo, iodo, O-alkylsulfonyl or O-arylsulfonyl, wherein aryl may be, for example, phenyl, naphthyl, or paranitrophenyl, by reacting them with an alkyl or arylsulfonylchloride in an appropriate solvent such as one selected from cyclic and acyclic mono and dialkylamides, chloroform, methylene chloride and pyridine and mixtures of the foregoing solvents at a temperature ranging from about -25°C to about 25°C, preferably at about -25°C, in presence of an acid acceptor an such as an alkali carbonate, a tertiary amine or a similar reagent. When L' in the desired compound of formula XIVA is chloro, bromo or iodo, the corresponding compound of formula XIV wherein R⁴ is methyl or trifluoromethyl is reacted with the appropriate phosphoryl or thionyl halide in a solvent such as chloroform, methylene chloride, benzene, toluene or mixtures thereof, at a temperature ranging from about 0°C to about the reflux temperature of the reaction mixture, preferably at the reflux temperature.

Compounds of the formula XIVA wherein R⁴ is methyl or trifluoromethyl and L' is a leaving group such as, for example, chloro, bromo, iodo, O-alkylsulfonyl and O-arylsulfonyl, wherein aryl may be, for example, phenyl, naphthyl, or paranitrophenyl, can be converted into compounds of the formula I by reacting said compound XIVA with a compound of general formula III. using similar solvents and conditions to those specified for the reaction shown in scheme 1.

These reactions can conveniently be carried out in solvents such as alcohols, cyclic and acyclic alkylketones, cyclic and acyclic alkylesters, cyclic and acyclic mono and dialkylamides, acetonitrile, and cyclic and acyclic alkyl ethers at a temperature ranging from 0°C to 150°C, preferable at 0°C or the boiling point of the same solvent. The presence of an acid acceptor such as an alkali carbonate, a tertiary amine or similar reagents may be useful.

The preparation of other compounds of the formula I not specifically described in the foregoing experimental section can be accomplished using combinations of the reactions described above that will be apparent to those skilled in the art.

In each of the reactions discussed or illustrated in schemes 1 to 4 above, pressure is not critical unless otherwise indicated. Pressures from about 0.5 atmospheres to about 4 atmospheres are generally acceptable, and ambient pressure, i.e., about 1 atmosphere, is preferred as a matter of convenience.

The novel compounds of the formula I and the pharmaceutically acceptable salts thereof (hereinafter "the therapeutic compounds of this invention") are useful as dopaminergic agents, i.e., they possess the ability to alter dopamine mediated neurotransmission in mammals, including humans. They are therefore able to function as therapeutic agents in the treatment of a variety of conditions in mammals, the treatment or prevention of which can be effected or facilitated by an increase or decrease in dopamine mediated neurotransmission. Such conditions include sleep disorders, sexual disorders (including sexual dysfunction), gastrointestinal disorders, psychosis, affective psychosis, nonorganic psychosis, personality disorders, psychiatric mood disorders, conduct and impulse disorders, schizophrenic and schizoaffective disorders, polydipsia, bipolar disorders, dysphoric mania, anxiety and related disorders, obesity, emesis, bacterial infections of the CNS such as meningitis, learning disorders, memory disorders, Parkinson's disease, depression, extrapyramidal side effects from neuroleptic agents, neuroleptic malignant syndrome, hypothalamic pituitary disorders, congestive heart failure, chemical dependencies such as drug and alcohol addictions, vascular and cardiovascular disorders, ocular disorders (including glaucoma), dystonia, tardive dyskinesia, Gilles De La Tourette's syndrome and other hyperkinesias, dementia, ischemia, Parkinson's disease, movement disorders such as akathesia, hypertension and diseases caused by a hyperactive immune system such as allergies and inflammation.

The compounds of the formula I that are basic in nature are capable of forming a wide variety of different salts with various inorganic and organic acids. Although such salts must be pharmaceutically acceptable for administration to animals, it is often desirable in practice to initially isolate a compound of the Formula I from the reaction mixture as a pharmaceutically unacceptable salt and then simply convert the latter back to the free base compound by treatment with an alkaline reagent and subsequently convert the latter free base to a pharmaceutically acceptable acid addition salt. The acid addition salts of the base compounds of this invention are readily prepared by treating the base compound with a substantially equivalent amount of the chosen mineral or organic acid in an aqueous solvent medium or in a suitable organic solvent, such as methanol or ethanol. Upon careful evaporation of the solvent, the desired solid salt is readily obtained. The desired acid salt can also be precipitated from a solution of the free base in an organic solvent by adding to the solution an appropriate mineral or organic acid.

The therapeutic compounds of this invention can be administered orally, transdermally (e.g., through the use of a patch), parenterally or topically. Oral administration is preferred. In general, these compounds are most desirably administered in dosages ranging from about 0.01 mg up to about 250 mg per day, although variations may occur depending on the weight and condition of the person being treated and the particular route of administration chosen. In some instances, dosage levels below the lower limit of the aforesaid range may be more than adequate, while in other cases still larger doses may be employed without causing any harmful side effect, provided that such larger doses are first divided into several small doses for administration throughout the day.

The therapeutic compounds of the invention may be administered alone or in combination with pharmaceutically acceptable carriers or diluents by either of the two routes previously indicated, and such administration may be carried out in single or multiple doses. More particularly, the novel therapeutic compounds of this invention can be administered in a wide variety of different dosage forms, i.e., they may be combined with various pharmaceutically acceptable inert carriers in the form of tablets, capsules, lozenges, troches, hard candies, powders, sprays, creams, salves, suppositories, jellies, gels, pastes, lotions, ointments, elixirs, syrups, and the like. Such carriers include solid diluents or fillers, sterile aqueous media and various non-toxic organic solvents, etc. Moreover, oral pharmaceutical compositions can be suitably sweetened and/or flavored.

For oral administration, tablets containing various excipients such as microcrystalline cellulose, sodium citrate, calcium carbonate, dicalcium phosphate and glycine may be employed along with various disintegrants such as starch (and preferably corn, potato or tapioca starch), alginic acid and certain complex silicates, together with granulation binders like polyvinylpyrrolidone, sucrose, gelatin and acacia. Additionally, lubricating agents such as magnesium stearate, sodium lauryl sulfate and talc are often very useful for tabletting purposes. Solid compositions of a similar type may also be employed as fillers in gelatin capsules; preferred materials in this connection also include lactose or milk sugar as well as high molecular weight polyethylene glycols. When aqueous suspensions and/or elixirs are desired for oral administration, the active ingredient may be combined with various sweetening or flavoring agents, coloring matter or dyes, and, if so desired, emulsifying and/or suspending agents as well, together with such diluents as water, ethanol, propylene glycol, glycerin and various like combinations thereof.

For parenteral administration, solutions of a compound of the present invention in either sesame or peanut oil or in aqueous propylene glycol may be employed. The aqueous solutions should be suitably buffered if necessary and the liquid diluent first rendered isotonic. These aqueous solutions are suitable for intravenous injection purposes. The oily solutions are suitable for intra-articular, intramuscular and subcutaneous injection purposes. The preparation of all these solutions under sterile conditions is readily accomplished by standard pharmaceutical techniques well known to those skilled in the art.

Additionally, it is also possible to administer the compounds of the present invention topically when treating inflammatory conditions of the skin and this may preferably be done by way of creams, jellies, gels, pastes, ointments and the like, in accordance with standard pharmaceutical practice.

The D4 dopaminergic activity of the compounds of the present invention may be determined by the following procedure.

The determination of D4 dopaminergic activity has been described by Van Tol et al., Nature, vol. 350, 610 (London, 1991). Clonal cell lines expressing the human dopamine D4 receptor are harvested and homogenized (teflon pestle) in a 50 mM Tris.HCI (pH 7.4 at 4°C) buffer containing 5 mM EDTA, 1.5 mM calcium chloride (CaCl₂), 5 mM magnesium chloride (MgCl₂), 5mM potassium chloride (KCI) and 120 mM sodium chloride (NaCI). The homogenates are centrifugated for 15 min. at 39,000 g, and the resulting pellets resuspended in a buffer at a concentration of 150-250 µg/ml. For saturation experiments, 0.25 ml aliquots of tissue homogenate are incubated in duplicate with increasing concentrations of [³H] Spiperone (70.3 Ci/mmol; 10-3000 pM final concentration) for 30-120 minutes at 22°C in a total volume of 1 ml. For competition binding experiments, assays are initiated by the addition of 0.25 ml of membrane and incubated in duplicate with the indicated concentrations of competing ligands (10⁻¹⁴-10⁻³ M) and [³H]Spiperone (100-300 pM) in either the absence or presence of 200 uM GPP(NH)^{P} (5'/guanylylimidodiphosphate), where indicated, for 60-120 min at 22°C. Assays are terminated by rapid filtration through a Titertek cell harvester and the filters subsequently monitored for tritium as described by Sunahara, R.K. et al., Nature, 346, 76-80 (1990). For all experiments, specific [³H]Spiperone binding is defined as that inhibited by 1-10 µM (+) Butaclamole or 1 µM Spiperone. Both saturation and competition binding data are analyzed by the non-linear least square curve-fitting program Ligand run on a digital Micro-PP-11 as described by Sunahara et al.

Approximately 80 compounds of the present invention were tested using the above procedure. All such compounds tested displaced [³H]-spiperone binding with a K of less than 1 µM.

The present invention is illustrated by the following examples. It will be understood, however, that the invention is not limited to the specific details of these examples. Melting points are uncorrected.

### EXAMPLE 1

### 1-(4-Fluoro-phenyl)-4-oxiranylmethyl-piperazine

A mixture of 2.5 gm of 1-(4-fluoro-phenyl)-piperazine, 3.0 gm of 2S-(+)glycidyl 3-nitrobenzensulfonate (available from Aldrich) and 15 ml dimethylformamide (DMF) was kept for 12 hours at ambient temperature. Thirty milliliters of water was added and the mixture was extracted with methylene chloride (CH₂Cl₂). The CH₂Cl₂ extract was collected, washed with 20 ml water and dried over sodium sulfate (Na₂SO₄). The crude product (3.5 gm of an oil) obtained after removing the solvents was purified by using chromatography: solid phase (SiO₂; 40µm: Baker); eluent 5% methanol (CH₃OH) in chloroform (CHCl₃). A sample of this purified material showed a M+/z of 236 and had an optical rotation of [α]_{D}=-15.54 (c=1.1, CHCl₃).

### EXAMPLE 2

### 1-Oxiranylmethyl-1H-benzoimidazole

A mixture of 2.7 gm of 1-H-benzoimidazole, and 1 gm sodiumhydride (60%) in 30 ml DMF was kept for 0.3 hours at ambient temperature. Six grams of 2R-(-) glycidyl 3-nitrobenzenesulfonate (available from Aldrich) was added and the mixture was stirred for 2 hours. The mixture was added to 100 ml ice and water and extracted with ethyl acetate. The ethyl acetate extract was collected, washed with 20 ml water and dried over Na₂SO₄. The crude product (6.5 gm of an oil) obtained after removing the solvents was purified by using chromatography: solid phase (silicon dioxide (SiO₂); 40µm; Baker); eluent 2% CH₃OH in CHCl₃. A sample of this purified material showed a M+/z of 175.

### EXAMPLE 3

### 1-Benzoimidazol-1-yl-3-[4-(4-fluoro-phenyl)-piperazin-1-yl]-propan-2-ol (both enantiomers)

A mixture of 0.26 gm of 1-H-benzoimidazole and 0.097 gm sodium hydride (60%) in 6 ml N,N-dimethylformamide (DMF) was kept for 0.3 hours at ambient temperature. 1-(4-fluoro-phenyl)-4-(-)oxiranylmethyl-piperazine (0.52 gm) was added and the mixture was stirred for 12 hours. This mixture was added to 10 ml ice and water and extracted with chloroform. The chloroform extract was collected, washed with 20 ml water and dried over Na₂SO₄. The crude product (1.0 gm of an oil) obtained after removing the solvents was purified using chromatography: solid phase (SiO₂; 40 µm; Baker); eluent 2% CH₃OH in CHCl₃. A sample of this purified material showed a M+/z of 355. It was transformed into its hydrochloride salt by treating an ethanolic suspension of this material with a mixture of ethyl ether/HCl. This salt had a melting point of 246-247°C and exhibited an optical rotation of [α]_{D}=-7.3 (c=0.5, MeOH).

A mixture of 2.0 gm of 1-oxiranylmethyl-1H-benzoimidazole, 2.37 gm of 1-(4-fluorophenyl)piperazine and 25 ml ethanol was stirred at 80°C for 48 hours. This mixture was added to 25 ml ice cold aqueous sodium hydroxide (NaOH) solution and extracted with CH₂Cl₂. The CH₂Cl₂ extract was collected, washed with 20 ml water and desiccated with Na₂SO₄. The crude product (94.18 gm of an oil) obtained after removal of solvents was purified using flash chromatography: solid phase (SiO₂; 40µM; Baker); eluent 5% CH₃OH in CH₂Cl₂. A sample of this purified material (2.14 gm) was transformed into its hydrochloride salt by treating an ethanolic suspension of this material with a mixture of ethyl ether/HCl. This salt had a melting point of 238-240°C and exhibited an optical rotation of [a]_{D}=-7.3 (c=0.5, MeOH).

### EXAMPLE 5

### N-(2-Nitro-4-fluorophenyl)-3-[4-4-fluorophenyl)piperazin-1-yl]propionamide

A mixture of 14.8 gm of N-(2-nitro-4-fluorophenyl)-3-bromo-propionamide, 9.0 gm of 1-(4-fluoro-phenyl)-piperazine, 6.46 gm of diisopropylethylamine and 150 ml DMF was kept for 48 hours at 150°C. Water (300 ml) was added at ambient temperature and the mixture was extracted with CHCl₃. The CHCl₃ extract was collected, washed with 20 ml water and dried over Na₂SO₄. The crude product obtained after removing the solvents was purified by using chromatography: solid phase (SiO₂; 40µm; Baker); eluent CHCl₃. A sample of this purified material (12.7 gm) exhibited the following data. M+/z of 405.

### EXAMPLE 6

### {3-[4-(4-Fluorophenyl)-piperazin-1-yl]-propyl}-(2-aminophenyl)amine

A mixture of 1.03 gm of N-(2-amino-4-fluorophenyl)-3-[4-(4-fluorophenyl)-piperazin-1-yl]-propionamide and 8.5 ml of a solution of 1.0 M lithium aluminum hydride in THF (added at a temperature of 9°C over a period of 0.3 hours) and 10 ml THF was kept for 12 hours at 20-25°C. Upon the slow addition of 1.7 ml 10% aqueous sodium hydroxide (NaOH), the mixture was treated with magnesium sulfate (MgSO₄) and the THF layer separated. The crude product, obtained after removing the solvent, was purified using standard chromatography: solid phase (SiO₂; 40µm; Baker); eluent 5% ethanol in ethyl acetate. A sample of this purified material (0.22 gm) exhibited the following data. M+/z of 361.

### EXAMPLE 7

### N-(2-Amino-4-fluorophenyl)-3-[4-(4-fluorophenyl)piperazin-1-yl]propionamide

A mixture of 3.0 gm of N-(2-nitro-4-fluorphenyl)-3-[4-(4 fluorophenyl)-piperazin-1-yl)-propionamide, 1 gm 5% palladium on carbon, 100 ml ethanol and 10 ml conc. hydrochloric acid was kept on a Parr Shaker in the presence of 45 psi hydrogen gas at 20-25°C. Upon cessation of hydrogen gas uptake, the mixture was purged with nitrogen and the ethanol layer separated. The crude product, obtained after removal of solvents, was purified by using standard chromatography: solid phase (SiO₂; 40µm; Baker) eluent 5% ethanol in ethyl acetate. A sample of this purified material (0.22 gm) exhibited the following data. M+/z of 375.

### EXAMPLE 8

### N-(2-Nitro-4-fluorophenyl)-3-bromopronionamide

A mixture of 7.8 gm of 4-fluoro-2-nitroaniline, 8.57 gm of 3-bromo propionylchloride (added at a temperature of 0°C over a period of 0.3 hours), 6.5 gm of diisopropylethylamine and 150 ml CH₂Cl₂ was kept for 48 hours at 20-25°C. Water (300 ml) was added and the mixture was extracted with CH₂Cl₂. The CH₂Cl₂ extract was collected, washed with water (2 x 20 ml) and desiccated wtih Na₂SO₄. The crude product (14.7 gm) obtained after removal of solvents was used without further purification. A sample of this crude material (14.79 gm) exhibited the following data. M+/z of 292.

### EXAMPLE 20

### 1-(5-Fluoro-2-methyl-benzoimidazol-1-yl)-3-[4-(4-fluoro-phenyl)-piperazin-1-yl]-propan-2-ol

### A. 1-(4-Fluoro-2-nitro-phenylamino)-3-[4-(4-fluoro-phenyl)-piperazin-1-yl]-propan-2-ol

A mixture of 2.84 gm of 1-(4-fluoro-phenyl)-4-oxiranylmethyl-piperazine; 2.6 ml of benzylamine and 25 ml ethanol is stirred at 80°C for 6 hours. This mixture is added to 25 ml ice-cold aqueous NaOH solution and extracted with CH₂Cl₂. The CH₂Cl₂ extract is collected, washed with 20 ml water and desiccated with Na₂SO₄. The crude product (4.18 gm of an oil) obtained after removal of solvents can be purified using flash chromatography: Solid phase: (SiO₂: 40µm; Baker); eluent: 5% CH₃OH in CH₂Cl₂. This material (3.23 gm) is debenzylated by stirring a methanolic solution (100 ml) of above material in the presence of ammonium formate (3.9 gm) and palladium on carbon (3.2 gm) for 12 hours. The corresponding primary amine is isolated by removing insoluble materials by filtration (celite) and concentrating the filtrate. Obtained is an oil that solidifies upon standing. A sample of this crude intermediate product (1.17 gm) is dissolved in 50 ml toluene and heated in the presence of 2,4-difluoro-nitrobenzene (0.5 gm) and potassium carbonate (0.7 gm) for 18 hours). The residue, obtained after removal of solvents, is partitioned between ethyl acetate (30 ml) and water (10 ml). The ethyl acetate layer is washed with water and brine (each 2 X 5 ml) and dried over anhydrous magnesium sulfate. The ethyl acetate layer is concentrated and the residue purified by flash chromatography on SiO₂ eluent ethyl acetate providing the intermediate (1 -(4-fluoro-2-nitro-phenylamino)-3-[4-(4-fluoro-phenyl)-piperazin-1-yl]-propan-2-ol.

### B. 1-(5-Fluoro-2-methyl-benzoimidazol-1-yl)-3-[4-(4-fluoro-phenyl)-piperazin-1-yl]-propan-2-ol

To a heated solution of 1-(4-fluoro-2-nitro-phenylamino)-3-[4-(4-fluoro-phenyl)-piperazin-1-yl]-propan-2-ol (0.73 gm) in acetic acid (20 ml) conaining acetic acid anhydride (0.4 ml) are added small portion of zinc dust (1.26 gm) over a period of 3 hours. Thereafter, the mixture is brought to ambient temperature, insoluble materials are removed by filtration (Celite®) and the filtrate is concentrated. This concentrate is partioned between ethyl acetate (50 ml) and aqueous sodium bicarbonate solution (30 ml). The ethyl acetate layer is washed with water and brine (each 2 X 10 ml) and dried over magnesium sulfate. Products are isolated by flash column chromatography, stationary phase: SiO₂ (40 µm, Baker); eluent: hexane followed by 50% ethyl acetate in hexane. Obtained are two products: (1) 1-(5-fluoro-2-methyl-benzoimidazol-1-yl)-3-[4-(4-fluoro-phenyl)-piperazin-1-yl]-propan-2-ol and (2) acetic acid 2-(5-fluoro-2-methylbenzoimidazol-1-yl)-1-[4-(4-fluoro-phenyl)-piperazin-1-ylmethyl]-ethyl ester. M.p. 108-110°C.

### EXAMPLE 23

### 5-Fluoro-1-{3-[4-(4-fluoro-phenyl)-piperazin-1-yl]-butyl}-2-methyl-1H-benzoimidazole

### A. 2-[4-(4-Fluoro-phenyl)-piperazin-1yl]-butyl}-propan-1-ol

A mixture of methanesulfonic acid 2-[(4-fluoro-phenyl)-(2-methanesulfonyloxy-ethyl)-amino]-ethyl ester prepared in situ from 9.1 gm of methansutfonic acid anhydride, 6.92 gm of 2-[(4-fluoro-phenyl)-(2-hydroxy-ethyl)-amino]-ethanol and 12.1 ml triethylamine and 50 ml methylenechloride) and 5.22 gm of (R)(-)2-amino 1-propanol in 20 ml ethanol is heated under reflux for 5 hours. The residue, obtained after removal of solvents, is dissolved in ethyl acetate (100 ml) and washed with water (2 x 20 ml). The ethyl acetate layer is concentrated and the products isolated by flash chromatography (solid phase: SiO₂; eluent: ethyl acetate). Obtained is a solid material (3.3 gm) consisting of 2-[4-(4-fluoro-phenyl)-piperazin-1-yl]-propan-1-ol.

### B. 3-[4-(4-Fluoro-phenyl)piperazin-1-yl]-butyronitrile

To a mixture of 2-[4-(4-fluoro-phenyl)-piperazin-1-yl]-propan-1-ol (1.7 gm) and 4-methylmorpholine (1.18 ml) in 6 ml chloroform is added 1.86 gm of methansulfonic acid anhydride in 5 ml chloroform at -15°C. The reaction is brought to ambient temperature after 30 minutes and t-butylammonium cyanide (9.6 gm) is added. After 12 hours solvents are removed and the residue partitioned between ethyl acetate water. The ethyl acetate layer is washed with water (4 x 20 ml), brine (2 x 10ml) and dried over sodium sulfate. The ethyl acetate layer is concentrated and the products isolated by flash chromatography (solid phase: SiO₂; eluent: 30% ethyl acetate in hexane). Obtained is an oily material (1.2 gm) consisting of 3-[4-(4-fluoro-phenyl)-piperazin-1-yl]-butyronitrile.

### C. (4-Fluoro-2-nitro-phenyl)-{3-[4-(4-fluoro-phenyl)-piperazin-1-yl]-butyl}-amine

3-[4-(4-fluoro-phenyl)-piperazin-1-yl]-butylamine. A mixture of 3-[4-(4-fluorophenyl)-piperazin-1-yl]-butyronitrile (0.84 gm), 0.811 gm of cobalt chloride (CoCl₂, 0.811 gm) and sodium borohydride (1.29 gm, added slowly over a period of 2 hours) in 35 ml methanol is stirred for 12 hours. Insoluble materials are removed by filtration and the filtrate is concentrated and the residue partitioned between ethyl acetate and 1N sodium hydroxide. The ethyl acetate layer is washed with water (4 x 20 ml), brine (2 x 10 ml) and dried *over* sodium sulfate. The ethyl acetate layer is concentrated and the crude product consisting of 3-[4-(4-fluoro-phenyl)-piperazin-1-yl]-butylamine (0.65 gm) is used as is.

3-[4-(4-Fluoro-phenyl)-piperazin-1-yl]-butylamine (0.65 gm) dissolved in 30 ml toluene is heated in the presence of 2,4-difluoro-nitrobenzene (0.49 gm) and potassium carbonate (0.43 gm) for 18 hours. The residue, obtained after removal of solvents, is partitioned between ethyl acetate (100 ml) and water (50 ml). The ethyl acetate layer is washed with water and brine (each 2 x 25 ml) and dried over anhydrous magnesum sulfate. The ethyl acetate layer is concentrated and products isolated by flash chromatography, (solid phase: SiO₂; eluent: gradient 10% ethyl acetate in hexane followed by 20% ethyl acetate in hexane), providing (4-fluoro-2-nitro-phenyl)-{3-[4-(4-fluoro-phenyl)-piperazin-1-yl]-butyl}-amine as an orange red colored solid.

### D. 5-Fluoro-1-{3-[4-(4-fluoro-phenyl)-piperazin-1-yl]-butyl}-2-methyl-1H-benzoimidazole

To a refluxing, stirred mixture of (4-fluoro-2-nitro-phenyl)-{3-[4-(4-fluorophenyl)-piperazin-1-yl]-butyl}-amine (0.38 gm), 10ml acetic acid are added small portions of Zn dust (excess) until a nearly colorless reaction mixture is obtained approx. 4 hours). Insolubles are removed by filtration, washed with acetic acid and the combined acetic acid layer concentrated to a black oil. This ally residue is dissolved in ethyl acetate (50 ml) and washed with 2 x 10 ml water and brine. The organic layer is collected, the sovent removed and the residue purified by flash chromatography (solid phase: SiO₂; eluent: gradient starting with ethyl acetate followed by ethyl acetate/methanol 8:2). Obtained are 0.1 gm of 5-fluoro-1-{3-[4-(4-fluoro-phenyl)-piperazin-1 -yl]-butyl}-2-methyl-1H-benzoimidazole. (M+/Z 384.47).

### EXAMPLE 24

### 5-Fluoro-1-{3-[4-(4-fluorophenyl)-piperazin-1-yl]-butyl}-1,3-dihydro-benzimidazol-2-one

The title compound was prepared using the procedure depicted In scheme 5. M+/Z 386. M.p. 188-193°C.

## Claims

1. A compound of the formula 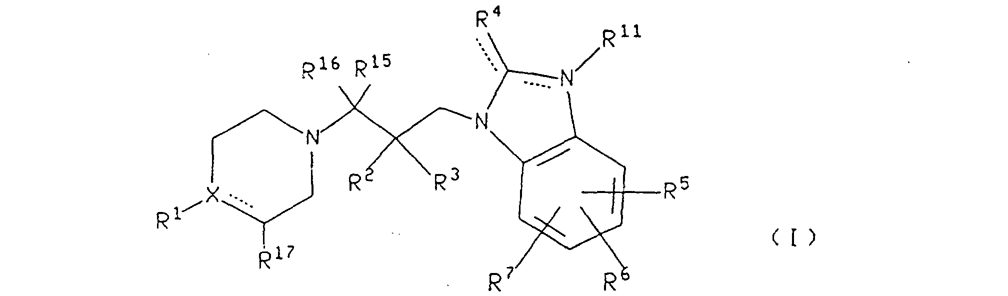 wherein each of the dotted lines represents an optional double bond;
X is carbon or nitrogen;
R¹ is (C₁-C₄) alkyl; aryl; where aryl is selected from phenyl, indanyl and naphthyl; or heteroaryl where heteroaryl is selected from pyridyl, thienyl, furyl, thiazolyl, isothiazolyl, oxazolyl, isoxazolyl, triazolyl, quinolyl and imidazolyl, wherein each of said foregoing (C₁-C₄), alkyl, aryl, and heteroaryl groups may optionally be substituted with one or more substituents independently selected from halo; (C₁-C₆) alkyl optionally substituted with from one to three flourine atoms, (C₁-C₆) alkoxy optionally substituted with from one to three flourine atoms, cyano, -C(=O)R⁸, aryl, wherein said aryl is independently selected from said above-recited aryl groups; and heteroaryl, wherein said heteroaryl is independently selected from said above-recited heteroaryl groups;
R² and R³ are independently selected from hydrogen, hydroxy, and (C₁-C₆) alkyl, provided at least one of R² and R³ is hydroxy;
R⁴ is hydrogen, sulfur, oxygen, (C₁-C₆) alkyl, amino, -NHR¹⁰, -SR¹⁰, or OR ¹⁰;
R⁵, R⁶ and R⁷ are independently selected from hydrogen, halo, cyano, (C₁-C₆)alkyl optionally substituted with from one to three fluorine atoms, (C₁-C₆)alkoxy optionally substituted with from one to three fluorine atoms, (C₁-C₆)alkylsulfonyl, (C₁-C₆)acylamino, (phenyl)[(C₁-C₆)acyl]amino, amino, (C₁-C₆)alkylamino, di-(C₁-C₆)alkylamino, aryl and heteroaryl, wherein said aryl is selected from phenyl, naphthyl and indanyl, and said heteroaryl is selected from pyridyl, thienyl, furyl, thiazolyl, isothiazolyl, oxazolyl, isoxazolyl, triazolyl, quinolyl and imidazolyl;
R⁸, R⁹ and R¹⁰ are independently selected from hydrogen and (C₁-C₆)alkyl; and
R¹¹ is hydrogen, (C₁-C₆)alkyl or benzyl, wherein the phenyl moiety of said benzyl may optionally be substituted with one or more substituents, preferably with from zero to two substituents, independently selected from halo, (C₁-C₆)alkyl optionally substituted with from one to three fluorine atoms, (C₁-C₆)alkoxy optionally substituted with from one to three fluorine atoms, amino, cyano, (C₁-C₆)alkylamino and di-(C₁-C₆)alkylamino;
each of R¹⁵ and R¹⁶ is selected, independently, from hydrogen, methyl, cyano, -(C=O)-NH₂ and -CH₂-O-(C₁-C₆)alkyl;
R¹⁷ is hydrogen or, when X is nitrogen, R¹⁷ may optionally form, together with the carbon to which it is attached, R¹ and X, a tetrahydroquinoline ring;
with the proviso that: (a) when the five membered ring of formula contains a double bond, R¹¹ is absent; (b) when R⁴ is sulfur or oxygen, R⁴ is double bonded to the carbon to which it is attached and such carbon is single bonded to both adjacent ring nitrogen atoms, and when R⁴ is hydroxyl, said carbon is double bonded to one said adjacent ring nitrogen atom as shown by said dotted line; (c) when X is nitrogen and is double bonded to an adjacent carbon, R¹ is absent; and (d) when R⁴ is methyl or oxygen, then both R¹⁵ and R¹⁶ are hydrogen.
or a pharmaceutically acceptable salt thereof.

2. A compound according to claim 1 wherein X is nitrogen.

3. A compound according to claim 1 wherein X is carbon.

4. A compound according to claim 2, wherein R¹ is absent.

5. A compound according to claim 1, wherein said compound is selected from the group consisting of:
1-Benzoimidazol-1-yl-3-[4-(4-fuoro-phenyl)-piperazin-1-yl]-propan-2-ol;
1-(5-Chloro-benzoimidazol-1-yl)-3-[4-(4-fluoro-phenyl)-piperazin-1-yl]-propan-2-ol;
1 -Benzoimidazol-1-yl-3-(4-o-tolyl-piperazine-1-yl)-propan-2-ol;
1 -Benzoimidazol-1-yl-3-(4-m-tolyl-piperazine-1-yl)-propan-2-ol;
1-Benzoimidazol-1-yl-3-(4-p-tolyl-piperazin-1-yl)-propan-2-ol;
1-Benzoimidazol-1-yl-3-{4-chloro-phenyl[-phenyl-methyl]-piperazin-1-yl}-propan-2-ol;
1-Benzoimidazol-1-yl-3-[4-(2-chloro-phenyl)-piperazin-1-yl]-propan-2-ol;
1-Benzoimidazol-1-yl-3-[4-(4-chloro-phenyl)-piperazin-1-yl]-propan-2-ol;
1-Benzoimidazol-1-yl-3-[4-(3-chloro-phenyl)-piperazin-1-yl]-propan-2-ol;
1-Benzoimidazol-1-yl-3-(4-pyrimidin-2-yl-piperazin-1-yl)-propan-2-ol;
1-Benzoimidazol-1-yl-3-(4-naphthalen-1-yl-piperazin-1-yl)-propan-2-ol;
1-Benzoimidazol-1-yl-3-[4-(3-trifluoromethyl-phenyl)-piperazin-1-yl]-propan-2-ol;
1-Benzoimidazol-1-yl-3-(4-benzyl-piperazin-1-yl)-propan-2-ol;
1-Benzoimidazol-1-yl-3-[4-(2-trifluoromethyl-benzyl)-piperazin-1-yl]-propan-2-ol;
1-Benzoimidazol-1-yl-3-[4-(2-ethoxy-benzyl)-piperazin-1-yl]-propan-2-ol;
1 -Benzoimidazol-1-yl-3-{4-{3-(3-trifluoromethyl-phenyl)-propyl]-piperazin-1-yl}-propan-2-ol; and
1-Benzoimidazol-1-yl-3-{4-[2-(3-trifluoromethyl-phenyl)-ethyl]-piperazin-1-yl}-propan-2-ol;

6. A pharmaceutical composition for use in treatment or prevention of a condition selected from sleep disorders, sexual disorders, gastrointestinal disorders, psychosis, affective psychosis, nonorganic psychosis, personality disorders, psychiatric mood disorders, conduct and impulse disorders, schizophrenic and schizoaffective disorders, polydipsia, bipolar disorders, dysphoric mania, anxiety and related disorders, obesity, emesis, bacterial infections of the CNS, learning disorders, memory disorders, Parkinson's disease, depression, extrapyramidal side effects from neuroleptic agents. neuroleptic malignant syndrome, hypothalamic pituitary disorders, ocular disorders. congestive heart failure, chemical dependencies, vascular and cardiovascular disorders. dystonia, tardive dyskinesia, Gilles De La Tourette's syndrome and other hyperkinesias. dementia, ischemia, Parkinson's disease, akathesia and other movement disorders. hypertension and diseases caused by a hyperactive immune system such as allergies and inflammation in a mammal, comprising an amount of a compound according to claim 1 that is effective in treating or preventing such condition, and a pharmaceutical acceptable carrier.

7. Use of a compound according to claim 1 in the preparation of a medicament that is effective in treating or preventing a condition selected from sleep disorders, sexual disorders, gastrointestinal disorders, psychosis, affective psychosis, nonorganic psychosis, personality disorders, psychiatric mood disorders, conduct and impulse disorders, schizophrenic and schizoaffective disorders, polydipsia, bipolar disorders, dysphoric mania, anxiety and related disorders, obesity, emesis, bacterial infections of the CNS, learning disorders, memory disorders, Parkinson's disease, depression, extrapyramidal side effects from neuroleptic agents, neuroleptic malignant syndrome, hypothalamic pituitary disorders, ocular disorders, congestive heart failure, chemical dependencies, vascular and cardiovascular disorders, dystonia, tardive dyskinesia, Gilles De La Tourette's syndrome and other hyperkinesias, dementia, ischemia, Parkinson's disease, akathesia and other movement disorders, hypertension and diseases caused by a hyperactive immune system such as allergies and inflammation in a mammal.

8. A pharmaceutical composition for use in treatment or prevention of a condition selected from sleep disorders, sexual disorders, gastrointestinal disorders, psychosis, affective psychosis, nonorganic psychosis, personality disorders, psychiatric mood disorders. conduct and impulse disorders, schizophrenic and schizoaffective disorders, polydipsia, bipolar disorders, dysphoric mania, anxiety and related disorders, obesity, emesis, bacterial infections of the CNS, learning disorders, memory disorders, Parkinson's disease, depression, extrapyramidal side effects from neuroleptic agents, neuroleptic malignant syndrome, hypothalamic pituitary disorders, ocular disorders, congestive heart failure, chemical dependencies, vascular and cardiovascular disorders, dystonia, tardive dyskinesia, Gilles De La Tourette's syndrome and other hyperkinesias, dementia, ischemia, Parkinson's disease, akathesia and other movement disorders, hypertension and diseases caused by a hyperactive immune system such as allergies and inflammation in a mammal, comprising a dopaminergic effective amount of a compound according to claim 1 and a pharmaceutically acceptable carrier.

9. Use of a dopaminergic effective amount of a compound according to claim 1 in the preparation of a medicament for treating or preventing a condition selected from sleep disorders, sexual disorders, gastrointestinal disorders, psychosis, affective psychosis, nonorganic psychosis, personality disorders, psychiatric mood disorders, conduct and impulse disorders, schizophrenic and schizoaffective disorders, polydipsia, bipolar disorders, dysphoric mania, anxiety and related disorders, obesity, emesis, bacterial infections of the CNS, learning disorders, memory disorders, Parkinson's disease, depression, extrapyramidal side effects from neuroleptic agents, neuroleptic malignant syndrome, hypothalamic pituitary disorders, ocular disorders, congestive heart failure, chemical dependencies, vascular and cardiovascular disorders, dystonia, tardive dyskinesia, Gilles De La Tourette's syndrome and other hyperkinesias, dementia, ischemia, Parkinson's disease, akathesia and other movement disorders, hypertension and diseases caused by a hyperactive immune system such as allergies and inflammation in a mammal.

10. A pharmaceutical composition for use in treatment or prevention of a disease or condition, the treatment or prevention of which can be effected or facilitated by altering dopamine mediated neurotransmission in a mammal, comprising a dopaminergic effective amount of a compound according to claim 1 and a pharmaceutically acceptable carrier.

11. Use of a dopaminergic effective amount of a compound according to claim 1 in the preparation of a medicament for treating or preventing a disease or condition, the treatment or prevention of which can be effected or facilitated by altering dopamine mediated neurotransmission in a mammal.

12. A pharmaceutical composition for use in treatment or prevention of a condition selected from sleep disorders, sexual disorders, gastrointestinal disorders, psychosis, affective psychosis, nonorganic psychosis, personality disorders, psychiatric mood disorders, conduct and impulse disorders, schizophrenic and schizoaffective disorders, polydipsia, bipolar disorders, dysphoric mania, anxiety and related disorders, obesity, emesis, bacterial infections of the CNS, leaming disorders, memory disorders, Parkinson's disease, depression, extrapyramidal side effects from neuroleptic agents, neuroleptic malignant syndrome, hypothalamic pituitary disorders, ocular disorders, congestive heart failure, chemical dependencies, vascular and cardiovascular disorders, dystonia, tardive dyskinesia, Gilles De La Tourette's syndrome and other hyperkinesias, dementia, ischemia, Parkinson's disease, akathesia and other movement disorders, hypertension and diseases caused by a hyperactive immune system such as allergies and inflammation in a mammal, comprising a D4 receptor binding effective amount of a compound according to claim 1 and a pharmaceutically acceptable carrier.

13. Use of a D4 receptor binding effective amount of a compound according to claim 1 in the preparation of a medicament for treating or preventing a condition selected from sleep disorders, sexual disorders, gastrointestinal disorders, psychosis, affective psychosis, nonorganic psychosis, personality disorders, psychiatric mood disorders, conduct and impulse disorders, schizophrenic and schizoaffective disorders, polydipsia, bipolar disorders, dysphoric mania, anxiety and related disorders, obesity, emesis, bacterial infections of the CNS, learning disorders, memory disorders, Parkinson's disease, depression, extrapyramidal side effects from neuroleptic agents, neuroleptic malignant syndrome, hypothalamic pituitary disorders, ocular disorders, congestive heart failure, chemical dependencies, vascular and cardiovascular disorders, dystonia, tardive dyskinesia, Gilles De La Tourette's syndrome and other hyperkinesias, dementia, ischemia, Parkinson's disease, akathesia and other movement disorders, hypertension and diseases caused by a hyperactive immune system such as allergies and inflammation in a mammal.

14. A pharmaceutical composition for use in treatment or prevention of a disease or condition, the treatment or prevention of which can be effected or facilitated by altering dopamine mediated neurotransmission in a mammal, comprising a D4 receptor binding effective amount of a compound according to claim 1 and a pharmaceutically acceptable carrier.

15. Use of a D4 receptor binding effective amount of a compound according to claim 1 in the preparation of a medicament for treating or preventing a disease or condition, the treatment or prevention of which can be effected or facilitated by altering dopamine mediated neurotransmission in a mammal.

16. A compound according to Claim 1 selected from
1-[4-(4-Fluoro-phenyl)-piperazin-1-yl]-3-(2-phenyl-benzoimidazol-1-yl)-propan-2-ol;
1-[4-(4-Fluoro-phenyl)-piperazin-1-yl]-3-(2-propyl-benzoimidazol-1-yl)-propan-2-ol;
1-[4-(4-Fluoro-phenyl)-piperazin-1-yl]-3-(2-methyl-benzoimidazol-1-yl)-propan-2-ol;
5-Chloro-1-(3-[4-(4-fluoro-phenyl)-piperazin-1-yl]-2-hydroxy-propyl)-1,3-dihydrobenzoimidazol-2-one;
5-Fluoro-1-(3-[4-(4-fluoro-phenyl)-piperazin-1-yl]-2-hydroxy-propyl)-1,3-dihydrobenzoimidazol-2-one;
1-Benzoimidazol-1-yl-3-[4-(3-methoxy-phenyl)-piperazin-1-yl]-propan-2-ol;
1-Benzoimidazol-1-yl-3-[4-(4-methoxy-phenyl)-piperazin-1-yl]propan-2-ol;
1-Benzoimidazol-1-yl-3-(4-phenyl-piperazin-1-yl)-propan-2-ol;
1-{4-[4-(3-Benzoimidazol-1-yl-2-hydroxy-propyl)-piperazin-1-yl]-phenyl}-ethanone;
1-(4-Benzo[1,3]dioxol-5-ylmethyl-piperazin-1-yl)-3-benzoimidazol-1-yl-propan-2-ol;
1-Benzoimidazol-1-yl-3-[4-(3,4-dimethyl-phenyl)-piperazin-1-yl]-propan-2-ol;
4-[4-(3-Benzoimidazol-1-yl-2-hydroxy-propyl)-piperazin-1-yl]-phenol;
1-Benzoimidazol-1-yl-3-(4-phenethyl-piperazin-1-yl)-propan-2-ol;
1-Benzoimidazol-1-yl-3-[4-(3-chloro-propyl)-piperazin-1-yl]-propan-2-ol;
2-[4-(3-Benzoimidazol-1-yl-2-hydroxy-propyl)-piperazin-1-yl-1-morpholin-4-yl-ethanone;
1-(4-Benzhydryl-piperazin-1-yl)-3-benzoimidazol-1-yl-propan-2-ol;
1-Benzoimidazol-1-yl-3-{4-[bis-(4-fluoro-phenyl)-methyl]-piperazin-1-yl}-propan-2-ol;
1-Benzoimidazol-1-yl-3-[4-(4-nitro-phenyl)-piperazin-1-yl]-propan-2-ol;
4-(3-Benzoimidazol-1-yl-2-hydroxy-propyl)-piperazine-1-carboxylic;
3-[4-(3-Benzoimidazol-1-yl-2-hydroxy-propyl)-piperazin-1-yl]-1-phenyl-propan-1-one;
4-[4-(3-Benzoimidazol-1-yl-2-hydroxy-propyl)-piperazin-1-yl]-1-(4-fluoro-phenyl)-butan-1-one;
1-Benzoimidazol-1-yl-3-[4-(tetrahydro-furan-2-ylmethyl)-piperazin-1-yl]-propan-2-ol;
[4-(3-Benzoimidazol-1-yl-2-hydroxy-propyl)-piperazin-1-yl]-(tetrahydro-furan-2-yl)-methanone;
[4-(3-Benzoimidazol-1 -yl-2-hydroxy-propyl)-piperazin-1-yl]-(2,3-dihydrobenzo[1,4]dioxin-2-yl)-methanone;
1-Benzoimidazol-1-yl-3-[4-(2,3-dihydro-benzo[1,4]dioxin-2-ylmethyl)-piperazin-1-yl]-propan-2-ol;
1-Benzoimidazol-1-yl-3-[4-2-vitro-butyl)-piperazin-1-yl]-propan-2-ol;
3-[4-(3-Benzoimidazol-1-yl-2-hydroxy-propyl)-piperazin-1-yl]-1-(4-chloro-phenyl)-propan-1-one;

## Patentansprüche

1. Verbindung der Formel 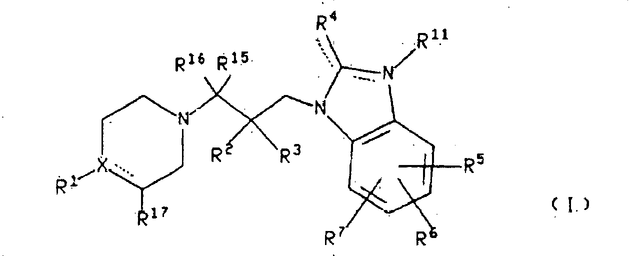 worin jede der Punktlinien eine mögliche Doppelbindung wiedergibt;
X Kohlenstoff oder Stickstoff darstellt;
R¹ (C₁-C₄)-Alkyl; Aryl; wobei Aryl ausgewählt ist aus Phenyl; Indanyl und Naphthyl; oder Heteroaryl, wobei Heteroaryl ausgewählt ist aus Pyridyl, Thienyl, Furyl, Thiazolyl, Isothiazolyl, Oxazolyl, Isoxazolyl, Triazolyl, Chinolyl und Imidazolyl, wobei jede der vorangehenden (C₁-C₄)-Alkyl-, Aryl- und Heteroarylgruppen gegebenenfalls mit einem oder mehreren Substituenten, unabhängig ausgewählt aus Halogen; (C₁-C₆)-Alkyl, gegebenenfalls substituiert mit einem bis drei Fluoratomen, (C₁-C₆)-Alkoxy, gegebenenfalls substituiert mit einem bis drei Fluoratomen, Cyano, -C(=O)R⁸, Aryl, worin das Aryl unabhängig ausgewählt ist aus den vorstehend angeführten Arylgruppen; und Heteroaryl, worin das Heteroaryl unabhängig ausgewählt ist aus den vorstehend angeführten Heteroarylgruppen, darstellt;
R² und R³ unabhängig ausgewählt sind aus Wasserstoff, Hydroxy und (C₁-C₆)-Alkyl, mit der Maßgabe, dass mindestens einer von R² und R³ Hydroxy darstellt;
R⁴ Wasserstoff, Schwefel, Sauerstoff, (C₁-C₆)-Alkyl, Amino, -NHR¹⁰, -SR¹⁰ oder -OR¹⁰ darstellt;
R⁵, R⁶ und R⁷ unabhängig ausgewählt sind aus Wasserstoff, Halogen, Cyano, (C₁-C₆)-Alkyl, gegebenenfalls substituiert mit einem bis drei Fluoratomen, (C₁-C₆)-Alkoxy, gegebenenfalls substituiert mit einem bis drei Fluoratomen, (C₁-C₆)-Alkylsulfonyl, (C₁-C₆)-Acylamino, (Phenyl)[(C₁-C₆)acyl]amino, Amino, (C₁-C₆)-Alkylamino, Di-(C₁-C₆)alkylamino, Aryl und Heteroaryl, wobei das Aryl aus Phenyl, Naphthyl und Indanyl ausgewählt ist, und das Heteroaryl aus Pyridyl, Thienyl, Furyl, Thiazolyl, Isothiazolyl, Oxazolyl, Isoxazolyl, Triazolyl, Chinolyl und Imidazolyl ausgewählt ist;
R⁸, R⁹ und R¹⁰ unabhängig aus Wasserstoff und (C₁-C₆)-Alkyl ausgewählt sind; und
R¹¹ Wasserstoff, (C₁-C₆)-Alkyl oder Benzyl darstellt, wobei die Phenyleinheit des Benzyls gegebenenfalls mit einem oder mehreren Substituenten, vorzugsweise null bis zwei Substituenten, unabhängig ausgewählt aus Halogen, (C₁-C₆)-Alkyl, gegebenenfalls substituiert mit einem bis drei Fluoratomen, (C₁-C₆)-Alkoxy, gegebenenfalls substituiert mit einem bis drei Fluoratomen, Amino, Cyano, (C₁-C₆)-Alkylamino und Di- (C₁-C₆) alkylamino, substituiert ist;
wobei jeder von R¹⁵ und R¹⁶ unabhängig aus Wasserstoff, Methyl, Cyano, - (C=O)-NH₂ und -CH₂-O-(C₁-C₆)-Alkyl ausgewählt ist;
R¹⁷ Wasserstoff darstellt oder, wenn X Stickstoff darstellt, R¹⁷ gegebenenfalls, zusammen mit dem Kohlenstoffatom, an das es gebunden ist, R¹ und X einen Tetrahydrochinolinring bilden kann;
mit der Maßgabe, dass: (a), wenn der fünfgliedrige Ring der Formel I eine Doppelbindung enthält, R¹¹ nicht vorliegt; (b), wenn R⁴ Schwefel oder Sauerstoff darstellt, R⁴ an das Kohlenstoffatom, an das es gebunden ist, doppelt gebunden vorliegt und ein solches Kohlenstoffatom an zwei benachbarte Ringstickstoffatome einfach gebunden vorliegt, und, wenn R⁴ Hydroxyl darstellt, der Kohlenstoff an das benachbarte Ringstickstoffatom doppelt gebunden ist, wie durch die Punktlinie gezeigt; (c), wenn X Stickstoff darstellt und an das benachbarte Kohlenstoffatom doppelt gebunden ist, R¹ nicht vorliegt; und (d), wenn R⁴ Methyl oder Sauerstoff darstellt, dann sowohl R¹⁵ als auch R¹⁶ Wasserstoff sind,
oder ein pharmazeutisch verträgliches Salz davon.

2. Verbindung nach Anspruch 1, worin X Stickstoff darstellt.

3. Verbindung nach Anspruch 1, worin X Kohlenstoff darstellt.

4. Verbindung nach Anspruch 2, worin R¹ nicht vorliegt.

5. Verbindung nach Anspruch 1, worin die Verbindung ausgewählt ist aus der Gruppe, bestehend aus:
1-Benzimidazol-1-yl-3-[4-(4-fluor-phenyl)-piperazin-1-yl]-propan-2-ol;
1-(5-Chlor-benzimidazol-1-yl)-3-[4-(4-fluor-phenyl)-piperazin-1-yl]-propan-2-ol;
1-Benzimidazol-1-yl-3-(4-o-tolyl-piperazin-1-yl)-propan-2-ol;
1-Benzimidazol-1-yl-3-(4-m-tolyl-piperazin-1-yl)-propan-2-ol;
1-Benzimidazol-1-yl-3-(4-p-tolyl-piperazin-1-yl)-propan-2-ol ;
1-Benzimidazol-1-yl-3-{4-chlor-phenyl[-phenyl-methyl]-piperazin-1-yl}-propan-2-ol;
1-Benzimidazol-1-yl-3-[4-(2-chlor-phenyl)-piperazin-1-yl]-propan-2-ol;
1-Benzimidazol-1-yl-3-[4-(4-chlor-phenyl)-piperazin-1-yl]-propan-2-ol;
1-Benzimidazol-1-yl-3-[4-(3-chlor-phenyl)-piperazin-1-yl]-propan-2-ol;
1-Benzimidazol-1-yl-3-(4-pyrimidin-2-yl-piperazin-1 yl)-propan-2-ol;
1-Benzimidazol-1-yl-3-(4-naphthalin-1-yl-piperazin-1-yl)-propan-2-ol;
1-Benzimidazol-1-yl-3-[4-(3-trifluormethyl-phenyl)-piperazin-1-yl]-propan-2-ol;
1-Benzimidazol-1-yl-3-(4-benzyl-piperazin-1-yl)-propan-2-ol;
1-Benzimidazol-1-yl-3-[4-(2-trifluormethyl-benzyl)-piperazin-1-yl]-propan-2-ol;
1-Benzimidazol-1-yl-3-[4-(2-ethoxy-benzyl)-piperazin-1-yl]-propan-2-ol;
1-Benzimidazol-1-yl-3-{4-[3-(3-trifluormethyl-phenyl)-propyl]-piperazin-1-yl}-propan-2-ol; und
1-Benzimidazol-1-yl-3-{4-[2-(3-tritfluormethyl-phenyl)-ethyl]-piperazin-1-yl} -propan-2-ol.

6. Pharmazeutische Zusammensetzung zur Verwendung bei der Behandlung oder Prävention eines Zustands, ausgewählt aus Schlafstörungen, Sexualstörungen, Gastrointestinalstörungen, Psychose, vorgetäuschter Psychose, nichtorganischer Psychose, Persönlichkeitsstörungen, psychiatrischen Stimmungsstörungen, Leitungs- und Impulsstörungen, schizophrenen und schizoaffektiven Störungen, Polydipsie, bipolaren Störungen, dysphorischer Manie, Angstzustand und verwandten Störungen, Fettsucht, Emesis, bakteriellen Infektionen des ZNS, Lernstörungen, Gedächtnisstörungen, Parkinson-Krankheit, Depression, außerordentlichen Nebenwirkungen von neuroleptischen Mitteln, neuroleptischem malignem Syndrom, hypothalamischen pituitären Störungen, Okularstörungen, Herzstauungsinsuffizienz, chemischen Abhängigkeiten, vaskulären und cardiovaskulären Störungen, Dystonie, tardiver Dyskinesie, Gilles-De-La-Tourette-Syndrom und anderen Hyperkinesien, Demenz, Ischämie, Parkinson-Krankheit, Akathesie und anderen Bewegungsstörungen, Hypertension und Krankheiten, die durch ein hyperaktives Immunsystem verursacht werden, wie Allergien und Entzündung, bei einem Säuger, umfassend eine bei der Behandlung oder Prävention eines solchen Zustands wirksame Menge einer Verbindung nach Anspruch 1 und einen pharmazeutisch verträglichen Träger.

7. Verwendung einer Verbindung nach Anspruch 1 bei der Herstellung eines Arzneimittels, das wirksam ist bei der Behandlung oder Prävention eines Zustands, ausgewählt aus Schlafstörungen, Sexualstörungen, Gastrointestinalstörungen, Psychose, vorgetäuschter Psychöse, nichtorganischer Psychose, Persönlichkeitsstörungen, psychiatrischen Stimmungsstörungen, Leitungs- und Impulsstörungen, schizophrenen und schizoaffektiven Störungen, Polydipsie, bipolaren Störungen, dysphorischer Manie, Angstzustand und verwandten Störungen, Fettsucht, Emesis, bakteriellen Infektionen des ZNS, Lernstörungen, Gedächtnisstörungen, Parkinson-Krankheit, Depression, außerordentlichen Nebenwirkungen von neuroleptischen Mitteln, neuroleptischem malignem Syndrom, hypothalamischen pituitären Störungen, Okularstörungen, Herzstauungsinsuffizienz, chemischen Abhängigkeiten, vaskulären und cardiovaskulären Störungen, Dystonie, tardiver Dyskinesie, Gilles-De-La-Tourette-Syndrom und anderen Hyperkinesien, Demenz, Ischämie, Parkinson-Krankheit, Akathesie und anderen Bewegungsstörungen, Hypertension und Krankheiten, die durch ein hyperaktives Immunsystem verursacht werden, wie Allergien und Entzündung, bei einem Säuger.

8. Pharmazeutische Zusammensetzung zur Verwendung bei der Behandlung oder Prävention eines Zustands, ausgewählt aus Schlafstörungen, Sexualstörungen, Gastrointestinalstörungen, Psychose, vorgetäuschter Psychose, nichtorganischer Psychose, Persönlichkeitsstörungen, psychiatrischen Stimmungsstörungen, Leitungs- und Impulsstörungen, schizophrenen und schizoaffektiven Störungen, Polydipsie, bipolaren Störungen, dysphorischer Manie, Angstzustand und verwandten Störungen, Fettsucht, Emesis, bakteriellen Infektionen des ZNS, Lernstörungen, Gedächtnisstörungen, Parkinson-Krankheit, Depression, außerordentlichen Nebenwirkungen von neuroleptischen Mitteln, neuroleptischem malignem Syndrom, hypothalamischen pituitären Störungen, Okularstörungen, Herzstauungsinsuffizienz, chemischen Abhängigkeiten, vaskulären und cardiovaskulären Störungen, Dystonie, tardiver Dyskinesie, Gilles-De-La-Tourette-Syndrom und anderen Hyperkinesien, Demenz, Ischämie, Parkinson-Krankheit, Akathesie und anderen Bewegungsstörungen, Hypertension und Krankheiten, die durch ein hyperaktives Immunsystem verursacht werden, wie Allergien und Entzündung, bei einem Säuger, umfassend eine dopaminerg wirksame Menge einer Verbindung nach Anspruch 1 und einen pharmazeutisch verträglichen Träger.

9. Verwendung einer dopaminerg wirksamen Menge einer Verbindung nach Anspruch, 1 bei der Herstellung eines Arzneimittels zur Behandlung oder Prävention eines Zustands, ausgewählt aus Schlafstörungen, Sexualstörungen, Gastrointestinalstörungen, Psychose, vorgetäuschter Psychose, nichtorganischer Psychose, Persönlichkeitsstörungen, psychiatrischen Stimmungsstörungen, Leitungs- und Impulsstörungen, schizophrenen und schizoaffektiven Störungen, Polydipsie, bipolaren Störungen, dysphorischer Manie, Angstzustand und verwandten Störungen, Fettsucht, Emesis, bakteriellen Infektionen des ZNS, Lernstörungen, Gedächtnisstörungen, Parkinson-Krankheit, Depression, außerordentlichen Nebenwirkungen von neuroleptischen Mitteln, neuroleptischem malignem Syndrom, hypothalamischen pituitären Störungen, Okularstörungen, Herzstauungsinsuffizienz, chemischen Abhängigkeiten, vaskulären und cardiovaskulären Störungen, Dystonie, tardiver Dyskinesie, Gilles-De-La-Tourette-Syndrom und anderen Hyperkinesien, Demenz, Ischämie, Parkinson-Krankheit, Akathesie und anderen Bewegungsstörungen, Hypertension und Krankheiten, die durch ein hyperaktives Immunsystem verursacht werden, wie Allergien und Entzündung, bei einem Säuger.

10. Pharmazeutische Zusammensetzung zur Verwendung bei der Behandlung oder Prävention einer Erkrankung oder eines Zustands, wobei die Behandlung oder Prävention davon durch Verändern der Dopamin-vermittelten Neurotransmission bei einem Säuger bewirkt oder erleichtert werden, kann, umfassend eine dopaminerg wirksame Menge einer Verbindung nach Anspruch 1 und einen pharmazeutisch verträglichen Träger.

11. Verwendung einer dopaminerg wirksamen Menge einer Verbindung nach Anspruch 1 bei der Herstellung eines Arzneimittels zur Behandlung oder Prävention einer Erkrankung oder eines Zustands, wobei die Behandlung oder Prävention davon durch Verändern der Dopamin-vermittelten Neurotransmission bei einem Säuger bewirkt oder erleichtert werden kann.

12. Pharmazeutische Zusammensetzung zur Verwendung bei der Behandlung oder Prävention eines Züstands, ausgewählt aus Schlafstörungen, Sexualstörungen, Gastrointestinalstörungen, Psychose, vorgetäuschter Psychose, nichtorganischer Psychose. Persönlichkeitsstörungen, psychiatrischen Stimmungsstörungen, Leitungs- und Impulsstörungen, schizophrenen und schizoaffektiven Störungen, Polydipsie, bipolaren Störungen, dysphorischer Manie, Angst zustand und verwandten Störungen, Fettsucht, Emesis, bakteriellen Infektionen des ZNS, Lernstörungen, Gedächtnisstörungen, Parkinson-Krankheit, Depression, außerordentlichen Nebenwirkungen von neuroleptischen Mitteln, neuroleptischem malignem Syndrom, hypothalamischen pituitären Störungen, Okularstörungen, Herzstauungsinsuffizienz, chemischen Abhängigkeiten, vaskulären und cardiovaskulären Störungen, Dystonie, tardiver Dyskinesie, Gilles-De-La-Tourette-Syndrom und anderen Hyperkinesien, Demenz, Ischämie, Parkinson-Krankheit, Akathesie und anderen Bewegungsstörungen, Hypertension und Krankheiten, die durch ein hyperaktives Immunsystem verursacht werden, wie Allergien und Entzündung, bei einem Säuger, umfassend eine wirksame, an D4-Rezeptor bindende Menge einer Verbindung nach Anspruch 1 und einen pharmazeutisch verträglichen Träger.

13. Verwendung einer wirksamen, an D4- Rezeptor bindenden Menge einer Verbindung nach Anspruch 1 bei der Herstellung eines Arzneimittels zur Behandlung oder Prävention eines Zustands, ausgewählt aus Schlafstörungen, Sexualstörungen, Gastrointestinalstörungen, Psychose, vorgetäuschter Psychose, nichtorganischer Psychose, Persönlichkeitsstörungen, psychiatrischen Stimmungsstörungen, Leitungs- und Impulsstörungen, schizophrenen und schizoaffektiven Störungen, Polydipsie, bipolaren Störungen, dysphorischer Manie, Angstzustand und verwandten Störungen, Fettsucht, Emesis, bakteriellen Infektionen des ZNS, Lernstörungen, Gedächtnisstörungen, Parkinson-Krankheit, Depression, außerordentlichen Nebenwirkungen von neuroleptischen Mitteln, neuroleptischem malignem Syndrom, hypothalamischen pituitären Störungen, Okularstörungen, Herzstauungsinsuffizienz, chemischen Abhängigkeiten, vaskulären und cardiovaskulären Störungen, Dystonie, tardiver Dyskinesie, Gilles-De-La-Tourette-Syndrom und anderen Hyperkinesien, Demenz, Ischämie, Parkinson-Krankheit , Akathesie und anderen Bewegungsstörungen, Hypertension und Krankheiten, die durch ein hyperaktives Immunsystem verursacht werden, wie Allergien und Entzündung, bei einem Säuger.

14. Pharmazeutische Zusammensetzung zur Verwendung bei der Behandlung oder Prävention einer Erkrankung oder eines Zustands, wobei die Behandlung oder Prävention davon durch Verändern der Dopamin-vermittelten Neurotransmission bei einem Säuger bewirkt oder erleichtert werden kann, umfassend eine wirksame, an D4-Rezeptor bindende Menge einer Verbindung nach Anspruch 1 und einen pharmazeutisch verträglichen Träger.

15. Verwendung einer wirksamen, an D4-Rezeptor bindenden Menge einer Verbindung nach Anspruch 1 bei der Herstellung eines Arzneimittels zur Behandlung oder Prävention einer Erkrankung oder eines Zustands, wobei die Behandlung oder Prävention davon durch Verändern der Dopamin-vermittelten Neurotransmission bei einem Säuger bewirkt oder erleichtert werden kann.

16. Verbindung nach Anspruch 1, ausgewählt aus
1-[4-(4-Fluor-phenyl) -piperazin-1-yl] -3-(2-phenyl-benzimidazol-1-yl)-propan-2-ol;
1-[4-(4-Fluor-phenyl)-piperazin-1-yl]-3-(2-propyl-benzimidazol-1-yl)-propan-2-ol;
1-[4-(4-Fluor-phenyl)-piperazin-1-yl]-3-(-2 methyl-benzimidazol-1-yl)-propan-2-ol;
5-Chlor-1-(3-[4-(4-fluor-phenyl)-piperazin-1-yl]-2-hydroxy-propyl)-1,3-dihydro-benzimidazol-2-on;
5-Fluor-1-(3-[4-(4-fluor-phenyl)-piperazin-1-yl]-2-hydroxy-propyl)-1,3-dihydro-benzimidazol-2-on;
1-Benzimidazol-1-yl-3-[4-(3-methoxy-phenyl)-piperazin-1-yl]-propan-2-ol;
1-Benzimidazol-1-yl-3-[4-(4-methoxy-phenyl)-piperazin-1-yl]-propan-2-ol;
1-Benzimidazol-1-yl-3-(4-phenyl-piperazin-1-yl)-propan-2-ol ;
1-{4- [4-(3-Benzimidazol-1-yl-2-hydroxy-propyl) -piperazin-1-yl]-phenyl}-ethanon;
1-(4-Benzo [1.3] dioxol-5-ylmethyl-piperazin-l-yl)-3-benzimidazol-1-yl-propan-2-ol;
1-Benzimidazol-1-yl-3-[4-(3,4-dimethyl-phenyl)-piperazin-1-yl]-propan-2-ol;
4-[4-(3-Benzimidazol-1-yl-2-hydroxy-propyl)-piperazin-1-yl]-phenol;
1-Benzimidazol-1-yl-3-(4-phenethyl-piperazin-1-yl)-propan-2-ol;
1-Benzimidazol-1-yl-3-[4-(3-chlor-propyl)-piperazin-1-yl]-propan-2-ol;
2-[4-(3-Benzimidazol-1-yl-2-hydroxy-propyl)-piperazin1-yl-1-morpholin-4-yl-ethanon;
1-(4-Benzhydryl-piperazin-1-yl)-3-benzimidazol-1-ylpropan-2-ol;
1-Benzimidazol-1-yl-3-{4-[bis-(4-fluor-phenyl)-methyl]-piperazin-1-yl}-propan-2-ol;
1-Benzimidazol-1-yl-3-[4-(4-nitro-phenyl)-piperazin-1-yl]-propan-2-ol;
4-(3-Benzimidazol-1-yl-2-hydroxy-propyl)-piperazin-1-carbonsäure;
3-[4-(3-Benzimidazol-1-yl-2-hydroxy-propyl)-piperazin-1-yl] -1-phenyl-propan-1-on;
4-[4-(3-Benzimidazol-1-yl-2-hydroxy-propyl)-piperazin-1-yl]-1-(4-fluor-phenyl)-butan-1-on;
1-Benzimidazol-1-yl-3-[4-(tetrahydro-furan-2-ylmethyl)-piperazin-1-yl]-propan-2-ol;
[4-(3-Benzimidazol-1-yl-2-hydroxy-propyl)-piperazin-1-yl]-(tetrahydro-furan-2-yl)-methanon;
[4-(3-Benzimidazol-1-yl-2-hydroxy-propyl)-piperazin-1-yl]-(2,3-dihydro-benzo[1.4] dioxin-2-yl)-methanon;
1-Benzimidazol-1-yl-3-[4-(2,3-dihydro-benzo [1.4]dioxin-2-ylmethyl)-piperazin-1-yl]-propan-2-ol;
1-Benzimidazol-1-yl-3-[4-(2-nitro-butyl)-piperazin-1-yl]-propan-2-ol;
3-[4-(3-Benzimidazol-1-yl-2-hydroxy-propyl)-piperazin-1-yl] -1-(4-chlor-phenyl)-propan-1-on.

## Revendications

1. Composé de formule 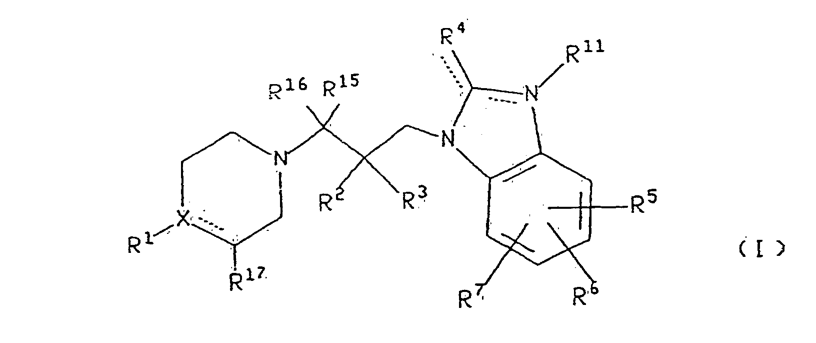 dans laquelle chacune des lignes discontinues représente une double liaison facultative ;
X représente un atome de carbone ou d'azote ;
R¹ représente un groupe alkyle en C₁ à C₄ ; aryle, le groupe aryle étant choisi entre les groupes phényle, indanyle et naphtyle ; ou hétéroaryle, le groupe hétéroaryle étant choisi entre les groupes pyridyle, thiényle, furyle, thiazolyle, isothiazolyle, oxazolyle, isoxazolyle, triazolyle, quinolyle et imidazolyle ; chacun desdits groupes alkyle en C₁ à C₄, aryle et hétéroaryle précités pouvant être facultativement substitué avec un ou plusieurs substituants choisis indépendamment entre des substituants halogéno, alkyle en C₁ à C₆ facultativement substitué avec un à trois atomes de fluor, alkoxy en C₁ à C₆ facultativement substitué avec un à trois atomes de fluor, cyano, -C(=O)R⁸, aryle, ledit groupe aryle étant choisi indépendamment parmi les groupes aryle précités, et hétéroaryle, ledit groupe hétéroaryle étant choisi indépendamment parmi lesdits groupes hétéroaryle précités ;
R² et R³ sont choisis indépendamment entre un atome d'hydrogène, un groupe hydroxy, un groupe alkyle en C₁ à C₆, sous réserve qu'au moins un des groupes R² et R³ représente un groupe hydroxy;
R⁴ représente un atome d'hydrogène, de soufre ou d'oxygène, un groupe alkyle en C₁ à C₆, amino, -NHR¹⁰, -SR¹⁰ ou -OR¹⁰;
R⁵, R⁶ et R⁷ sont choisis indépendamment entre un atome d'hydrogène, des groupes halogèno, cyano, alkyle en C₁ à C₆ facultativement substitué avec un à trois atomes de fluor, alkoxy en C₁ à C₆ facultativement substitué avec un à trois atomes de fluor, alkylsulfonyle en C₁ à C₆, acylamino en C₁ à C₆, (phényle) (acyle en C₁ à C₆) amino, amino, alkylamino en C₁ à C₆, di(alkyle en C₁ à C₆) amino, aryle et hétéroaryle, ledit groupe aryle étant choisi entre les groupes phényle, naphtyle et indanyle, et ledit groupe hétéroaryle étant choisi entre les groupes pyridyle, thiényle, furyle, thiazolyle, isothiazolyle, oxazolyle, isoxazolyle, triazolyle, quinolyle et imidazolyle ;
R⁸, R⁹ et R¹⁰ sont choisis indépendamment entre un atome d'hydrogène et un groupe alkyle en C₁ à C₆ ; et
R¹¹ représente un atome d'hydrogène, un groupe alkyle en C₁ à C₆ ou benzyle, le groupement phényle dudit groupe benzyle pouvant être facultativement substitué avec un ou plusieurs substituants, de préférence avec 0 à 2 substituants, choisis indépendamment entre des substituants halogéno, alkyle en C₁ à C₆ facultativement substitué avec un à trois atomes de fluor, alkoxy en C₁ à C₆ facultativement substitué avec un à trois atomes de fluor, amino, cyano, alkylamino en C₁ à C₆ et di(alkyle en C₁ à C₆) amino ;
chacun des groupes R¹⁵ et R¹⁶ est choisi, indépendamment, entre un atome d'hydrogène, des groupes méthyle, cyano, -(C=O)-NH₂ et -CH₂-O- (alkyle en C₁ à C₆) ;
R¹⁷ représente un atome d'hydrogène ou, lorsque X représente un atome d'azote, R¹⁷ peut former facultativement, conjointement avec l'atome de carbone auquel il est fixé, R¹ et X, un noyau tétrahydroquinoléine;
sous réserve que : (a) lorsque le noyau pentagonal de la formule I contient une double liaison, R¹¹ soit absent; (b) lorsque R⁴ représente un atome de soufre ou d'oxygène, R⁴ soit doublement lié à l'atome de carbone auquel il est fixé et cet atome de carbone soit lié par une liaison simple aux deux atomes d'azote adjacents du noyau et, lorsque R⁴ représente un groupe hydroxyle, ledit atome de carbone soit doublement lié audit atome d'azote adjacent du noyau, de la manière représentée par ladite ligne discontinue ;(c) lorsque X représente un atome d'azote et est doublement lié à un atome de carbone adjacent, R¹ soit absent ; et (d) lorsque R⁴ représente un groupe méthyle ou un atome d'oxygène, alors R¹⁵ et R¹⁶ représentent tous deux un atome d'hydrogène ;
ou un de ses sels pharmaceutiquement acceptables.

2. Composé suivant la revendication 1, dans lequel X représente un atome d'azote.

3. Composé suivant la revendication 1, dans lequel X représente un atome de carbone.

4. Composé suivant la revendication 2, dans lequel R¹ est absent.

5. Composé suivant la revendication 1, ledit composé étant choisi dans le groupe consistant en :
1-benzimidazole-1-yl-3-[4-(4-fluoro-phenyl)-pipérazine-1-yl]-propane-2-ol;
1-(5-chloro-benzimidazole-1-yl-)-3-[4-(4-fluorophényl)-pipérazine-1-yl]-propane-2-ol;
1-benzimidazole-1-yl-3-(4-o-tolyl-pipérazine-1-yl)-propane-2-ol;
1-benzimidazole-1-yl-3-(4-m-tolyl-pipérazine-1-yl) -propane-2-ol ;
1-benzimidazole-1-yl-3-(4-p-tolyl-pipérazine-1-yl)-propane-2-ol;
1-benzimidazole-1-yl-3-{4-chloro-phényl-[phényl-méthyl] -pipérazine-1-yl}-propane-2-ol ;
1-benzimidazole-1-yl-3-[4-(2-chloro-phényl)-pipérazine-1-yl]-propane-2-ol ;
1-benzimidazole-1-yl-3-[4-(4-chloro-phényl)-pipérazine-1-yl]-propane-2-ol ;
1-benzimidazole-1-yl-3-[4-(3-chloro-phényl)-pipérazine-1-yl]-propane-2-ol;
1-benzimidazole-1-yl-3-(4-pyrimidin-2-yl-pipérazine-1-yl)-propane-2-ol ;
1-benzimidazole-1-yl-3-(4-naphtalène-1-yl-pipérazine-1-yl) -propane-2-ol ;
1-benzimidazole-1-yl-3-[4-(3-trifluorométhylphényl)-pipérazine-1-yl]-propane-2-ol ;
1-benzimidazole-1-yl-3-(4-benzyl-pipérazine-1-yl)-propane-2-ol ;
1-benzimidazole-1-yl-3-[4-(2-trifluorométhylbenzyl)-pipérazine-1-yl]-propane-2-ol ;
1-benzimidazole-1-yl-3-[4-(2-éthoxy-benzyl)-pipérazine-1-yl]-propane-2-ol ;
1-benzimidazole-1-yl-3-{4-{3-(3-trifluorométhylphényl)-propyl]-pipérazine-1-yl}-propane-2-ol ;et
1-benzimidazole-1-yl-3-{4-[2-(3-trifluorométhylphényl)-éthyl]-pipérazine-1-yl}-propane-2-ol.

6. Composition pharmaceutique destinée à être utilisée dans le traitement ou la prévention d'une affection choisie entre des troubles du sommeil, des troubles sexuels, des troubles gastro-intestinaux, la psychose, la psychose affective, la psychose non organique, des troubles de la personnalité, des troubles psychiatriques de l'humeur, des troubles de la conduite et d'impulsivité, des troubles schizophréniques et schizo-affectifs, la polydipsie, des troubles bipolaires, la manie dysphorique, l'anxiété et des troubles apparentés, l'obésité, les vomissements, des infections bactériennes du SNC, des troubles de l'apprentissage, des troubles de la mémoire, la maladie de Parkinson, la dépression, les effets secondaires extrapyramidaux des agents neuroleptiques, le syndrome malin produit par les agents neuroleptiques, des troubles hypothalamo-hypophysaires, des troubles oculaires, l'insuffisance cardiaque congestive, des dépendances chimiques, des troubles vasculaires et cardiovasculaires, la dystonie, la dyskinésie tardive, le syndrome de Gilles De La Tourette et d'autres hyperkinésies, la démence, l'ischémie, la maladie de Parkinson, l'acathésie et d'autres troubles du mouvement, l'hypertension et des maladies provoquées par un système immunitaire hyperactif telles que des allergies et l'inflammation chez un mammifère, comprenant une quantité d'un composé suivant la revendication 1 qui est efficace dans le traitement ou la prévention d'une telle affection, et un support pharmaceutiquement acceptable.

7. Utilisation d'un composé suivant la revendication 1 dans la préparation d'un médicament qui est efficace dans le traitement- ou la prévention d'une affection choisie entre des troubles du sommeil, des troubles sexuels, des troubles gastro-intestinaux, la psychose, la psychose affective, la psychose non organique, des troubles de la personnalité, des troubles psychiatriques de l'humeur, des troubles de la conduite et d'impulsivité, des troubles schizophréniques et schizo-affectifs, la polydipsie, des troubles bipolaires, la manie dysphorique, l'anxiété et des troubles apparentés, l'obésité, les vomissements, des infections bactériennes du SNC, des troubles de l'apprentissage, des troubles de la mémoire, la maladie de Parkinson, la dépression, les effets secondaires extrapyramidaux des agents neuroleptiques, le syndrome malin produit par les agents neuroleptiques, des troubles hypothalamo-hypophysaires, des troubles oculaires, l'insuffisance cardiaque congestive, des dépendances chimiques, des troubles vasculaires et cardiovasculaires, la dystonie, la dyskinésie tardive, le syndrome de Gilles De La Tourette et d'autres hyperkinésies, la démence, l'ischémie, la maladie de Parkinson, l'acathésie et d'autres troubles du mouvement, l'hypertension et des maladies provoquées par un système immunitaire hyperactif telles que des allergies et l'inflammation chez un mammifère.

8. Composition pharmaceutique destinée à être utilisée dans le traitement ou la prévention d'une affection choisie entre des troubles du sommeil, des troubles sexuels, des troubles gastro-intestinaux, la psychose, la psychose affective, la psychose non organique, des troubles de la personnalité, des troubles psychiatriques de l'humeur, des troubles de la conduite et d'impulsivité, des troubles schizophréniques et schizo-affectifs, la polydipsie, des troubles bipolaires, la manie dysphorique, l'anxiété et des troubles apparentés, l'obésité, les vomissements, des infections bactériennes du SNC, des troubles de l'apprentissage, des troubles de la mémoire, la maladie de Parkinson, la dépression, les effets secondaires extrapyramidaux des agents neuroleptiques, le syndrome malin produit par les agents neuroleptiques, des troubles hypothalamo-hypophysaires, des troubles oculaires, l'insuffisance cardiaque congestive, des dépendances chimiques, des troubles vasculaires et cardiovasculaires, la dystonie, la dyskinésie tardive, le syndrome de Gilles De La Tourette et d'autres hyperkinésies, la démence, l'ischémie, la maladie de Parkinson, l'acathésie et d'autres troubles du mouvement, l'hypertension et des maladies provoquées par un système immunitaire hyperactif telles que des allergies et l'inflammation chez un mammifère, comprenant, une quantité, efficace du point de vue dopaminergique, d'un composé suivant la revendication 1 et un support pharmaceutiquement acceptable.

9. Utilisation d'une quantité, efficace du point de vue dopaminergique, d'un composé suivant la revendication 1, dans la préparation d'un médicament destiné au traitement ou à la prévention d'une affection choisie entre des troubles du sommeil, des troubles sexuels, des troubles gastro-intestinaux, la psychose, la psychose affective, la psychose non organique, des troubles de la personnalité, des troubles psychiatriques de l'humeur, des troubles de la conduite et d'impulsivité, des troubles schizophréniques et schizo-affectifs, la polydipsie, des troubles bipolaires, la manie dysphorique, l'anxiété et des troubles apparentés, l'obésité, les vomissements, des infections bactériennes du SNC, des troubles de l'apprentissage, des troubles de la mémoire, la maladie de Parkinson, la dépression, les effets secondaires extrapyramidaux des agents neuroleptiques, le syndrome malin produit par les agents neuroleptiques, des troubles hypothalamo-hypophysaires, des troubles oculaires, l'insuffisance cardiaque congestive, des dépendances chimiques, des troubles vasculaires et cardiovasculaires, la dystonie, la dyskinésie tardive, le syndrome de Gilles De La Tourette et d'autres hyperkinésies, la démence, l'ischémie, la maladie de Parkinson, l'acathésie et d'autres troubles du mouvement, l'hypertension et des maladies provoquées par un système immunitaire hyperactif telles que des allergies et l'inflammation chez un mammifère.

10. Composition pharmaceutique destinée à être utilisée dans le traitement ou la prévention d'une maladie ou affection, dont le traitement ou la prévention peut être effectué ou facilité en modifiant la neurotransmission à médiation par la dopamine chez un mammifère, comprenant une quantité, efficace du point de vue dopaminergique, d'un composé suivant la revendication 1 et un support pharmaceutiquement acceptable.

11. Utilisation d'une quantité, efficace du point de vue dopaminergique, d'un composé suivant la revendication 1 dans la préparation d'un médicament destiné au traitement ou à la prévention d'une maladie ou affection, dont le traitement ou la prévention peut être effectué ou facilité en modifiant la neurotransmission à médiation par la dopamine chez un mammifère.

12. Composition pharmaceutique destinée à être utilisée dans le traitement ou la prévention d'une affection choisie entre des troubles du sommeil, des troubles sexuels, des troubles gastro-intestinaux, la psychose, la psychose affective, la psychose non organique, des troubles de la personnalité, des troubles psychiatriques de l'humeur, des troubles de la conduite et d'impulsivité, des troubles schizophréniques et schizo-affectifs, la polydipsie, des troubles bipolaires, la manie dysphorique, l'anxiété et des troubles apparentés, l'obésité, les vomissements, des infections bactériennes du SNC, des troubles de l'apprentissage, des troubles de la mémoire, la maladie de Parkinson, la dépression, les effets secondaires extrapyramidaux des agents neuroleptiques, le syndrome malin produit par les agents neuroleptiques, des troubles hypothalamo-hypophysaires, des troubles oculaires, l'insuffisance cardiaque congestive, des dépendances chimiques, des troubles vasculaires et cardiovasculaires, la dystonie, la dyskinésie tardive, le syndrome de Gilles De La Tourette et d'autres hyperkinésies, la démence, l'ischémie, la maladie de Parkinson, l'acathésie et d'autres troubles du mouvement, l'hypertension et des maladies provoquées par un système immunitaire hyperactif telles que des allergies et l'inflammation chez un mammifère, comprenant une quantité, efficace pour se lier aux récepteurs D4, d'un composé suivant la revendication 1 et un support pharmaceutiquement acceptable.

13. Utilisation d'une quantité, efficace pour se lier aux récepteurs D4, d'un composé suivant la revendication 1, dans la préparation d'un médicament destiné au traitement ou à la prevention d'une affection choisie entre des troubles du sommeil, des troubles sexuels, des troubles gastro-intestinaux, la psychose, la psychose affective, la psychose non organique, des troubles de la personnalité, des troubles psychiatriques de l'humeur, des troubles de la conduite et d'impulsivité, des troubles schizophréniques et schizo-affectifs, la polydipsie, des troubles bipolaires, la manie dysphorique, l'anxiété et des troubles apparentés, l'obésité, les vomissements, des infections bactériennes du SNC, des troubles de l'apprentissage, des troubles de la mémoire, la maladie de Parkinson, la dépression, les effets secondaires extrapyramidaux des agents neuroleptiques, le syndrome malin produit par les agents, neuroleptiques, des troubles hypothalamo-hypophysaires, des troubles oculaires, l'insuffisance cardiaque congestive, des dépendances chimiques, des troubles vasculaires et cardiovasculaires, la dystonie, la dyskinésie tardive, le syndrome de Gilles De La Tourette et d'autres hyperkinesies, la démence, l'ischémie, la maladie de Parkinson, l'acathésie et d'autres troubles du mouvement, l'hypertension et des maladies provoquées par un système immunitaire hyperactif telles que des allergies et l'inflammation chez un mammifère.

14. Composition pharmaceutique destinée à être utilisée dans le traitement ou la prévention d'une maladie ou affection, dont le traitement ou la prévention peut être effectué ou facilité en modifiant la neurotransmission à médiation par la dopamine chez un mammifère, comprenant une quantité, efficace pour se lier aux récepteurs D4, d'un composé suivant la revendication 1 et un support pharmaceutiquement acceptable.

15. Utilisation d'une quantité, efficace pour se lier aux récepteurs D4, d'un composé suivant la revendication 1, dans la préparation d'un médicament destiné au traitement ou à la prévention d'une maladie ou affection, dont le traitement ou la prévention peut être effectué ou facilité en modifiant la neurotransmission à médiation par la dopamine chez un mammifère.

16. Composé suivant la revendication 1, choisi entre les suivants :
1-[4-(4-fluoro-phényl)-pipérazine-1-yl]-3-(2-phényl-benzimidazole-1-yl)-propane-2-ol;
1-[4-(4-fluoro-phényl)-pipérazine-1-yl]-3-(2-propyl-benzimidazole-1-yl)-propane-2-ol;
1-[4-(4-fluoro-phényl)-pipérazine-1-yl]-3-(2-méthyl-benzimidazole-1-yl)-propane-2-ol;
5-chloro-1-(3-[4-(4-fluorophényl)-pipérazine-1-yl]-2-hydroxypropyl)-1,3-dihydro-benzimidazole-2-one;
5-fluoro-1-(3-[4-(4-fluorophényl)-pipérazine-1-yl]-2-hydroxypropyl)-1,3-dihydro-benzimidazole-2-one;
1-benzimidazole-1-yl-3-[4-(3-méthoxyphényl)-pipérazine-1-yl]-propane-2-ol;
1-benzimidazole-1-yl-3-[4-(4-méthoxyphényl)-pipérazine-1-yl]-propane-2-ol;
1-benzimidazole-1-yl-3-(4-phényl-pipérazine-1-yl] -propane-2-ol ;
1-{4-[4-(3-benzimidazole-1-yl-2-hydroxypropyl)-pipérazine-1-yl]-phényl}-éthanone ;
1- (4-benzo [1,3] dioxol-5-ylméthyl-pipérazine-1-yl)-3-benzimidazole-1-yl-propane-2-ol ;
1-benzimidazole-1-yl-3-[4-(3,4-diméthylphényl)-pipérazine-1-yl]-propane-2-ol ;
4-[4-(3-benzimidazole-1-yl-2-hydroxypropyl)-pipérazine-1-yl] -phénol ;
1-benzimidazole-1-yl-3-(4-phénétyl-pipérazine-1-yl) -propane-2-ol ;
1-benzimidazole-1-yl-3-[4-(3-chloropropyl)-pipérazine-1-yl]-propane-2-ol ;
2-[4-(3-benzimidazole-1-yl-2-hydroxypropyl)-pipérazine-1-yl-1-morpholine-4-yl-éthanone ;
1-(4-benzhydryl-pipérazine-1-yl)-3-benzimidazole-1-yl-propane-2-ol ;
1-benzimidazole-1-yl-3-{4- [bis- (4-fluorophényl)-méthyl]-pipérazine-1-yl}-propane-2-ol ;
1-benzimidazole-1-yl-3-[4-(4-nitrophényl)-pipérazine-1-yl]-propane-2-ol ;
4-(3-benzimidazole-1-yl-2-hydroxy-propyl)-pipérazine-1-carboxylique;
3-[4-(3-benzimidazole-1-yl-2-hydroxy-propyl)-pipérazine-1-yl]-1-phényl-propane-1-one ;
4-[4-(3-benzimidazole-1-yl-2-hydroxy-propyl)-pipérazine-1-yl] -1- (4-fluoro-phényl) -butan-1-one ;
1-benzimidazole-1-yl-3-[4-(tétrahydrofuranne-2-ylméthyl)-pipérazine-1-yl]propane-2-ol;
[4-(3-benzimidazole-1-yl-2-hydroxy-propyl)-pipérazine-1-yl]-(tétrahydrofuranne-2-yl)-méthanone;
[4-(3-benzimidazole-1-yl-2-hydrox-propyl)-pipérazine-1-yl]-(2,3-dihydro-benzo[1,4]dioxine-2-yl)-méthanone;
1-benzimidazole-1-yl-3-[4-(2,3-dihydrobenzo [1,4] dioxin-2-ylméthyl)-pipérazine-1-yl]-propane-2-ol ;
1-benzimidazole-1-yl-3-[4-(2-nitrobutyl)-pipérazine-1-yl]-propane-2-ol ;
3- [4- (3-benzimidazole-1-yl-2-hydroxypropyl)-pipérazine-1-yl]-1-(4-chloro-phényl)-propane-1-one.
